# EUROPEAN PATENT SPECIFICATION

(11) **EP 3 941 545 B1**
(45) Date of publication and mention of the grant of the patent: **14.05.2025**
(21) Application number: 20711541.1
(22) Date of filing: 10.03.2020
(51) Int. Cl.: A61M 1/00, A61F 13/05

(54) **EXHAUST BLOCKAGE DETECTION FOR NEGATIVE PRESSURE WOUND TREATMENT APPARATUSES**
ERKENNUNG VON ABGASVERSTOPFUNGEN FÜR UNTERDRUCKWUNDBEHANDLUNGSGERÄTE
DÉTECTION D'UNE OBSTRUCTION DE L'ÉVACUATION POUR APPAREILS DE TRAITEMENT DES PLAIES PAR PRESSION NÉGATIVE

(30) Priority: 20.03.2019 GB 201903778
(43) Date of publication of application: 26.01.2022
(73) Proprietor: Smith & Nephew plc, Watford, Hertfordshire WD18 8YE (GB)
(72) Inventor: RODRIGUES, Roberto, Damiao Da Costa, Hull HU3 2BN (GB)
(74) Representative: Guy, Mark Robert
(86) International application number: PCT/EP2020/056317
(87) International publication number: WO 2020/187641

(56) References cited:
- US-A1- 2011 071 483
- US-A1- 2015 057 625
- US-A1- 2016 136 339
- US-A1- 2017 112 974
- US-A1- 2017 354 768
- US-B2- 7 569 742

## Description

### TECHNICAL FIELD

Embodiments described herein relate to apparatuses, systems, and methods (not claimed) for the treatment of wounds, using dressings in combination with negative pressure wound therapy. Examples of prior art systems are described e.g. in US2017/112974A1 and US2011/071483.

### DESCRIPTION OF THE RELATED ART

The treatment of open or chronic wounds that are too large to spontaneously close or otherwise fail to heal by means of applying negative pressure to the site of the wound is well known in the art. Negative pressure wound therapy (NPWT) systems currently known in the art commonly involve placing a cover that is impermeable or semi-permeable to fluids over the wound, using various means to seal the cover to the tissue of the patient surrounding the wound, and connecting a source of negative pressure (such as a vacuum pump) to the cover in a manner so that negative pressure is created and maintained under the cover. It is believed that such negative pressures promote wound healing by facilitating the formation of granulation tissue at the wound site and assisting the body's normal inflammatory process while simultaneously removing excess fluid, which may contain adverse cytokines and/or bacteria. However, further improvements in NPWT are needed to fully realize the benefits of treatment.

### SUMMARY

The invention is defined by the appended claims.

The methods do not form part of the claimed invention.

A negative pressure wound therapy system can include a wound dressing configured to be placed over a wound, the wound dressing configured to absorb fluid, a source of negative pressure disposed on or within the dressing, the source of negative pressure configured to aspirate fluid from the wound, a vent configured to allow gas aspirated by the source of negative pressure to flow outside the system, a pressure sensor at least partially disposed on or within the dressing proximal to the vent, and a controller disposed on or within the dressing, the controller configured to, in response to a determination that pressure measured by the pressure sensor satisfies a threshold indicative of blockage in the vent, provide an indication that the vent is blocked.

The system of any preceding paragraphs and/or any of the systems disclosed herein can include one or more of the following features. The system can include a chamber at least partially disposed on or within the dressing, the chamber housing the pressure sensor and comprising the vent through a wall of the chamber. The controller can be further configured to set the threshold to a value associated with an initial pressure measured by the pressure sensor prior to activation of the source of negative pressure. The controller can be further configured to adjust the set threshold by a positive pressure offset. The controller can be further configured to periodically activate the source of negative pressure to establish a target negative pressure under the dressing and, in response to establishing the target negative pressure under the dressing, deactivate the source of negative pressure. The controller can be further configured to, in response to a determination that pressure measured by the pressure sensor following an activation of the source of negative pressure satisfies the threshold, provide the indication that the vent is blocked. The controller can be configured to determine if the pressure measured by the pressure sensor satisfies the threshold following each activation of the source of negative pressure.

The system of any preceding paragraphs and/or any of the systems disclosed herein can include one or more of the following features. The system can further include another pressure sensor configured to measure pressure in a fluid flow path connecting the source of negative pressure to the wound, and the controller can be further configured to, based on pressure measured by the another pressure sensor, determine if a level of fluid absorbed by the wound dressing satisfies a threshold fluid level and provide another indication in response to a determination that the level of fluid absorbed by the wound dressing satisfied the threshold fluid level. The threshold fluid level can be indicative of the wound dressing being substantially full. The system can further comprise a filter positioned upstream of the source of negative pressure and configured to substantially prevent passage of fluid downstream of the filter, and the controller can be further configured to determine blockage of the filter and provide another indication associated with the blockage. The system can further comprise at least one indicator positioned at least partially on an exterior surface of the wound dressing, and the controller can be configured to provide at least one of the indication or another indication via the at least one indicator.

A negative pressure wound therapy system can include a source of negative pressure configured to aspirate fluid from a wound covered by a wound dressing, a first pressure sensor configured to measure pressure downstream of the source of negative pressure, and a controller configured to, in response to a determination that pressure measured by the first pressure sensor satisfies a threshold indicative of a first blockage downstream of the source of negative pressure, provide an indication of the first blockage.

The system of any preceding paragraphs and/or any of the systems disclosed herein can include one or more of the following features. The system can further comprise a vent configured to release gas aspirated by the source of negative pressure into the atmosphere, and the first blockage can be associated with blockage of the vent. The system can further comprise an enclosure comprising a vent in communication with the atmosphere, and the first sensor can be positioned in the enclosure. The threshold can be indicative of atmospheric pressure adjusted by a positive pressure offset. The controller can be further configured to detect and provide an indication of a second blockage upstream of the source of negative pressure.

The system of any preceding paragraphs and/or any of the systems disclosed herein can include one or more of the following features. The system can further comprise a second pressure sensor configured to measure pressure upstream of the source of negative pressure, and the controller can be further configured to, in response to a determination that pressure measured by the second pressure sensor satisfies a threshold indicative of a second blockage upstream of the source of negative pressure, provide an indication of the second blockage. The second blockage can be associated with blockage in a fluid flow path connecting the source of negative pressure to the wound dressing. The system can further comprise at least one indicator visible to a user, and the controller can be configured to provide at least one of the indications of the first or second blockages via the at least one indicator.

One or more methods of using and/or operating the negative pressure wound therapy system of any preceding paragraphs or any of the systems disclosed herein can be provided.

A method of operating a negative pressure wound therapy system can include, by a pressure sensor, measuring pressure at one or more of 1) proximal to an exhaust of the system, the exhaust configured to allow gas aspirated from a wound to be expelled outside the system or 2) downstream of a source of negative pressure of the system, the source of negative pressure configured to aspirate fluid from the wound and, by a controller of the system, in response to determining that the measured pressure satisfies a threshold indicative one or more of 1) a blockage in the exhaust or 2) a blockage downstream of the source of negative pressure, indicating the blockage.

The method of any preceding paragraphs and/or any of the methods disclosed herein can include one or more of the following features. The threshold can correspond to a value associated with an initial pressure measured by the pressure sensor prior to activation of the source of negative pressure and, optionally, the value can be adjusted by a positive pressure offset. The method can further comprise, by the controller, activating the source of negative pressure to establish a target negative pressure under a dressing of the system, the dressing configured to be placed over the wound and, following activation of the source of negative pressure, providing the indication of the blockage in response to determining that the measured pressure satisfies the threshold.

### BRIEF DESCRIPTION OF THE DRAWINGS

Figures 1A-1C illustrates a wound dressing incorporating the source of negative pressure and/or other electronic components within the wound dressing;
Figures 2A-2C illustrate embodiments of an electronics unit incorporated into a wound dressing;
Figure 3A illustrates an embodiment of wound dressing layers incorporating the electronic components within the wound dressing;
Figure 3B illustrates a cross sectional layout of the material layers of the wound dressing incorporating an electronics assembly within the dressing;
Figure 3C illustrates a top view of an embodiment of the wound dressing incorporating an electronic assembly within the dressing;
Figures 4A and 4B illustrate an embodiment of a housing of the electronics assembly enclosing the electronics unit within;
Figures 5A-5B illustrate embodiments of the electronics assembly positioned within an aperture in wound dressing layers;
Figure 6 is an exploded perspective view of an embodiment of an electronics assembly enclosing an electronics unit within a housing;
Figure 7A illustrates a bottom perspective view of the electronics assembly of Figure 6;
Figure 7B illustrates a top perspective view of the electronics assembly of Figure 6;
Figures 7C-7D show embodiments of an upper surface of an electronics assembly;
Figure 8 illustrates an embodiment of wound dressing layers of a wound dressing for use with an electronics assembly;
Figure 9 illustrates an embodiment of a wound dressing incorporating an electronics assembly within the wound dressing layers;
Figures 10A-10B and 11A-11B illustrate embodiments of components of an electronics assembly;
Figure 12 illustrates an embodiment of a pump outlet mechanism;
Figure 13 illustrates a diagram of fluid flow according to an embodiment; and
Figure 14 illustrates a flow chart for detecting a downstream blockage according to an embodiment.

### DETAILED DESCRIPTION

Embodiments disclosed herein relate to apparatuses and methods of treating a wound with reduced pressure, including a source of negative pressure and wound dressing components and apparatuses. The apparatuses and components comprising the wound overlay and packing materials, if any, are sometimes collectively referred to herein as dressings.

It will be appreciated that throughout this specification reference is made to a wound. It is to be understood that the term wound is to be broadly construed and encompasses open and closed wounds in which skin is torn, cut or punctured or where trauma causes a contusion, or any other superficial or other conditions or imperfections on the skin of a patient or otherwise that benefit from reduced pressure treatment. A wound is thus broadly defined as any damaged region of tissue where fluid may or may not be produced. Examples of such wounds include, but are not limited to, abdominal wounds or other large or incisional wounds, either as a result of surgery, trauma, sterniotomies, fasciotomies, or other conditions, dehisced wounds, acute wounds, chronic wounds, subacute and dehisced wounds, traumatic wounds, flaps and skin grafts, lacerations, abrasions, contusions, burns, diabetic ulcers, pressure ulcers, stoma, surgical wounds, trauma and venous ulcers or the like.

It will be understood that embodiments of the present disclosure are generally applicable to use in topical negative pressure ("TNP") therapy systems. Briefly, negative pressure wound therapy assists in the closure and healing of many forms of "hard to heal" wounds by reducing tissue oedema; encouraging blood flow and granular tissue formation; removing excess exudate and may reduce bacterial load (and thus infection risk). **In** addition, the therapy allows for less disturbance of a wound leading to more rapid healing. TNP therapy systems may also assist on the healing of surgically closed wounds by removing fluid and by helping to stabilize the tissue in the apposed position of closure. A further beneficial use of TNP therapy can be found in grafts and flaps where removal of excess fluid is important and close proximity of the graft to tissue is required in order to ensure tissue viability.

As is used herein, reduced or negative pressure levels, such as -X mmHg, represent pressure levels relative to normal ambient atmospheric pressure, which can correspond to 760 mmHg (or 1 atm, 29.93 inHg, 101.325 kPa, 14.696 psi, 1013.25 mbar, etc.). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than X mmHg corresponds to pressure that is closer to atmospheric pressure (such as,-40 mmHg is less than -60 mmHg). Negative pressure that is "more" or "greater" than -X mmHg corresponds to pressure that is further from atmospheric pressure (such as, -80 mmHg is more than -60 mmHg). In some embodiments, local ambient atmospheric pressure is used as a reference point, and such local atmospheric pressure may not necessarily be, for example, 760 mmHg.

The negative pressure range for some embodiments of the present disclosure can be approximately -80 mmHg, or between about -20 mmHg and -200 mmHg. Note that these pressures are relative to normal ambient atmospheric pressure, which can be 760 mmHg. Thus, -200 mmHg would be about 560 mmHg in practical terms. In some embodiments, the pressure range can be between about -40 mmHg and -150 mmHg. Alternatively a pressure range of up to -75 mmHg, up to -80 mmHg or over -80 mmHg can be used. Also in other embodiments a pressure range of below -75 mmHg can be used. Alternatively, a pressure range of over approximately -100 mmHg, or even -150 mmHg, can be supplied by the negative pressure apparatus.

In some embodiments of wound closure devices described herein, increased wound contraction can lead to increased tissue expansion in the surrounding wound tissue. This effect may be increased by varying the force applied to the tissue, for example by varying the negative pressure applied to the wound over time, possibly in conjunction with increased tensile forces applied to the wound via embodiments of the wound closure devices. In some embodiments, negative pressure may be varied over time for example using a sinusoidal wave, square wave, and/or in synchronization with one or more patient physiological indices (such as, heartbeat). Examples of such applications where additional disclosure relating to the preceding may be found include U.S. Patent No. 8,235,955, titled "Wound treatment apparatus and method," issued on August 7, 2012; and U.S. Patent No. 7,753,894, titled "Wound cleansing apparatus with stress," issued July 13, 2010. The disclosures of both of these patents are hereby incorporated by reference in their entirety.

International Application PCT/GB2012/000587, titled "WOUND DRESSING AND METHOD OF TREATMENT" and filed on July 12, 2012, and published as WO 2013/007973 A2 on January 17, 2013, is an application that is directed to embodiments, methods of manufacture, and wound dressing components and wound treatment apparatuses that may be used in combination or in addition to the embodiments described herein. Additionally, embodiments of the wound dressings, wound treatment apparatuses and methods described herein may also be used in combination or in addition to those described in International Application No. PCT/IB2013/001469, filed May 22, 2013, titled "APPARATUSES AND METHODS FOR NEGATIVE PRESSURE WOUND THERAPY," published as WO 2013/175306 on November 28, 2013, US Patent Application No. 14/418874, filed January 30, 2015, published as U.S. Publication No. 2015/0216733, published August 6, 2015, titled "WOUND DRESSING AND METHOD OF TREATMENT," U.S. Patent Application No. 14/418908, filed January 30, 2015, published as U.S. Publication No. 2015/0190286, published July 9, 2015, titled "WOUND DRESSING AND METHOD OF TREATMENT," U.S. Patent Application No. 14/658,068, filed March 13, 2015, U.S. Application No. 2015/0182677, published July 2, 2015, titled "WOUND DRESSING AND METHOD OF TREATMENT". Embodiments of the wound dressings, wound treatment apparatuses and methods described herein may also be used in combination or in addition to those described in U.S. Patent Application No. 13/092,042, filed April 21 2011, published as U.S. 2011/0282309, titled "WOUND DRESSING AND METHOD OF USE", including further details relating to embodiments of wound dressings, the wound dressing components and principles, and the materials used for the wound dressings.

Embodiments of the wound dressings, wound treatment apparatuses and methods described herein relating to wound dressings with electronics incorporated into the dressing may also be used in combination or in addition to those described in PCT Application Number PCT/EP2017/055225, filed March 6, 2017, titled "WOUND TREATMENT APPARATUSES AND METHODS WITH NEGATIVE PRESSURE SOURCE INTEGRATED INTO WOUND DRESSING," including further details relating to embodiments of wound dressings, the wound dressing components and principles, and the materials used for the wound dressings.

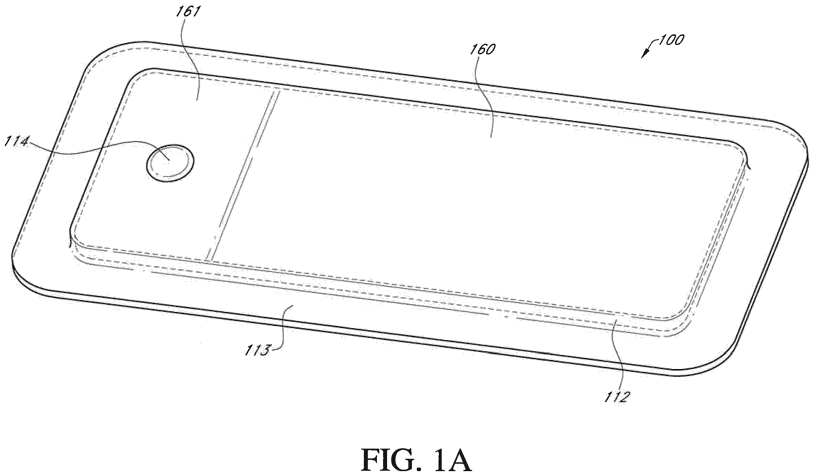

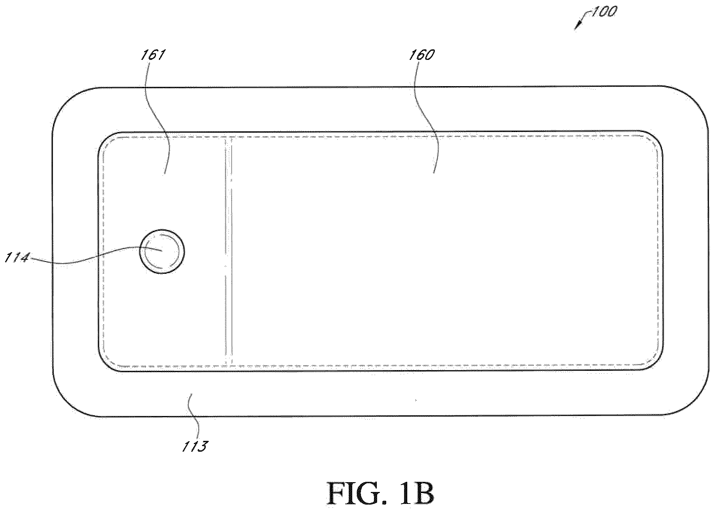

In some embodiments, a source of negative pressure (such as a pump) and some or all other components of the TNP system, such as power source(s), sensor(s), connector(s), user interface component(s) (such as button(s), switch(es), speaker(s), screen(s), etc.) and the like, can be integral with the wound dressing. The wound dressing can include various material layers described here and described in further detail in International Application No. PCT/EP2017/055225, filed March 6, 2017, entitled WOUND TREATMENT APPARATUSES AND METHODS WITH NEGATIVE PRESSURE SOURCE INTEGRATED INTO WOUND DRESSING. The material layers can include a wound contact layer, one or more absorbent layers, one or more transmission or spacer layers, and a backing layer or cover layer covering the one or more absorbent and transmission or spacer layers. The wound dressing can be placed over a wound and sealed to the wound with the pump and/or other electronic components contained under the cover layer within the wound dressing. In some embodiments, the dressing can be provided as a single article with all wound dressing elements (including the pump) pre-attached and integrated into a single unit. In some embodiments, a periphery of the wound contact layer can be attached to the periphery of the cover layer enclosing all wound dressing elements as illustrated in Figure 1A-1C.

In some embodiments, the pump and/or other electronic components can be configured to be positioned adjacent to or next to the absorbent and/or transmission layers so that the pump and/or other electronic components are still part of a single article to be applied to a patient. In some embodiments, the pump and/or other electronics can be positioned away from the wound site. Figures 1A-1C illustrates a wound dressing incorporating the source of negative pressure and/or other electronic components within the wound dressing. Figures 1A-1C illustrates a wound dressing 100 with the pump and/or other electronics positioned away from the wound site. The wound dressing can include an electronics area 161 and an absorbent area 160. The dressing can comprise a wound contact layer 110 (not shown in Figures 1A-1B) and a moisture vapor permeable film or cover layer 113 positioned above the contact layer and other layers of the dressing. The wound dressing layers and components of the electronics area as well as the absorbent area can be covered by one continuous cover layer 113 as shown in Figures 1A-1C.

The dressing can comprise a wound contact layer 110, a transmission layer 111, an absorbent layer 112, a moisture vapor permeable film or cover layer 113, 113 positioned above the wound contact layer, transmission layer, absorbent layer, or other layers of the dressing. The wound contact layer can be configured to be in contact with the wound. The wound contact layer can include an adhesive on the patient facing side for securing the dressing to the surrounding skin or on the top side for securing the wound contact layer to a cover layer or other layer of the dressing. In operation, the wound contact layer can be configured to provide unidirectional flow so as to facilitate removal of exudate from the wound while blocking or substantially preventing exudate from returning to the wound.

The wound contact layer 110 can be a polyurethane layer or polyethylene layer or other flexible layer which is perforated, for example via a hot pin process, laser ablation process, ultrasound process or in some other way or otherwise made permeable to liquid and gas. The wound contact layer 110 has a lower surface and an upper surface. The perforations preferably comprise through holes in the wound contact layer 110 which enable fluid to flow through the layer 110. The wound contact layer 110 helps prevent tissue ingrowth into the other material of the wound dressing. Preferably, the perforations are small enough to meet this requirement while still allowing fluid to flow therethrough. For example, perforations formed as slits or holes having a size ranging from 0.025 mm to 1.2 mm are considered small enough to help prevent tissue ingrowth into the wound dressing while allowing wound exudate to flow into the dressing. In some configurations, the wound contact layer 110 may help maintain the integrity of the entire dressing 100 while also creating an air tight seal around the absorbent pad in order to maintain negative pressure at the wound.

Some embodiments of the wound contact layer 110 may also act as a carrier for an optional lower and upper adhesive layer (not shown). For example, a lower pressure sensitive adhesive may be provided on the lower surface of the wound dressing 100 whilst an upper pressure sensitive adhesive layer may be provided on the upper surface of the wound contact layer. The pressure sensitive adhesive, which may be a silicone, hot melt, hydrocolloid or acrylic based adhesive or other such adhesives, may be formed on both sides or optionally on a selected one or none of the sides of the wound contact layer. When a lower pressure sensitive adhesive layer is utilized it may be helpful to adhere the wound dressing 100 to the skin around a wound site. In some embodiments, the wound contact layer may comprise perforated polyurethane film. The lower surface of the film may be provided with a silicone pressure sensitive adhesive and the upper surface may be provided with an acrylic pressure sensitive adhesive, which may help the dressing maintain its integrity. In some embodiments, a polyurethane film layer may be provided with an adhesive layer on both its upper surface and lower surface, and all three layers may be perforated together.

A layer 111 of porous material can be located above the wound contact layer 110. As used herein, the terms porous material, spacer, and/or transmission layer can be used interchangeably to refer to the layer of material in the dressing configured to distribute negative pressure throughout the wound area. This porous layer, or transmission layer, 111 allows transmission of fluid including liquid and gas away from a wound site into upper layers of the wound dressing. In particular, the transmission layer 111 preferably ensures that an open air channel can be maintained to communicate negative pressure over the wound area even when the absorbent layer has absorbed substantial amounts of exudates. The layer 111 should preferably remain open under the typical pressures that will be applied during negative pressure wound therapy as described above, so that the whole wound site sees an equalized negative pressure. The layer 111 may be formed of a material having a three dimensional structure. For example, a knitted or woven spacer fabric (for example Baltex 7970 weft knitted polyester) or a non-woven fabric could be used.

The transmission layer assists in distributing negative pressure over the wound site and facilitating transport of wound exudate and fluids into the wound dressing. In some embodiments, the transmission layer can be formed at least partially from a three dimensional (3D) fabric.

In some embodiments, the transmission layer 111 comprises a 3D polyester spacer fabric layer including a top layer (that is to say, a layer distal from the wound-bed in use) which is a 84/144 textured polyester, and a bottom layer (that is to say, a layer which lies proximate to the wound bed in use) which is a 10 denier flat polyester and a third layer formed sandwiched between these two layers which is a region defined by a knitted polyester viscose, cellulose or the like monofilament fiber. Other materials and other linear mass densities of fiber could of course be used.

Whilst reference is made throughout this disclosure to a monofilament fiber it will be appreciated that a multistrand alternative could of course be utilized. The top spacer fabric thus has more filaments in a yarn used to form it than the number of filaments making up the yarn used to form the bottom spacer fabric layer.

This differential between filament counts in the spaced apart layers helps control moisture flow across the transmission layer. Particularly, by having a filament count greater in the top layer, that is to say, the top layer is made from a yarn having more filaments than the yarn used in the bottom layer, liquid tends to be wicked along the top layer more than the bottom layer. In use, this differential tends to draw liquid away from the wound bed and into a central region of the dressing where the absorbent layer 112 helps lock the liquid away or itself wicks the liquid onwards towards the cover layer 113 where it can be transpired.

Preferably, to improve the liquid flow across the transmission layer 111 (that is to say perpendicular to the channel region formed between the top and bottom spacer layers), the 3D fabric may be treated with a dry cleaning agent (such as, but not limited to, Perchloro Ethylene) to help remove any manufacturing products such as mineral oils, fats or waxes used previously which might interfere with the hydrophilic capabilities of the transmission layer. In some embodiments, an additional manufacturing step can subsequently be carried in which the 3D spacer fabric is washed in a hydrophilic agent (such as, but not limited to, Feran Ice 30g/l available from the Rudolph Group). This process step helps ensure that the surface tension on the materials is so low that liquid such as water can enter the fabric as soon as it contacts the 3D knit fabric. This also aids in controlling the flow of the liquid insult component of any exudates.

Further, an absorbent layer (such as layer 112) for absorbing and retaining exudate aspirated from the wound can be utilized. In some embodiments, a superabsorbent material can be used in the absorbent layer 112. In some embodiments, the absorbent includes a shaped form of a superabsorber layer.

A layer 112 of absorbent material is provided above the transmission layer 111. The absorbent material, which comprise a foam or non-woven natural or synthetic material, and which may optionally comprise a super-absorbent material, forms a reservoir for fluid, particularly liquid, removed from the wound site. In some embodiments, the layer 111 may also aid in drawing fluids towards the cover layer 113.

The material of the absorbent layer 112 may also prevent liquid collected in the wound dressing from flowing freely within the dressing, and preferably acts so as to contain any liquid collected within the dressing. The absorbent layer 112 also helps distribute fluid throughout the layer via a wicking action so that fluid is drawn from the wound site and stored throughout the absorbent layer. This helps prevent agglomeration in areas of the absorbent layer. The capacity of the absorbent material must be sufficient to manage the exudates flow rate of a wound when negative pressure is applied. Since in use the absorbent layer experiences negative pressures the material of the absorbent layer is chosen to absorb liquid under such circumstances. A number of materials exist that are able to absorb liquid when under negative pressure, for example superabsorber material. The absorbent layer 112 may typically be manufactured from ALLEVYN^{™} foam, Freudenberg 114-224-4 or Chem-Posite^{™}11C-450. In some embodiments, the absorbent layer 112 may comprise a composite comprising superabsorbent powder, fibrous material such as cellulose, and bonding fibers. In a preferred embodiment, the composite is an airlaid, thermally-bonded composite.

In some embodiments, the absorbent layer 112 is a layer of non-woven cellulose fibers having super-absorbent material in the form of dry particles dispersed throughout. Use of the cellulose fibers introduces fast wicking elements which help quickly and evenly distribute liquid taken up by the dressing. The juxtaposition of multiple strand-like fibers leads to strong capillary action in the fibrous pad which helps distribute liquid. In this way, the super-absorbent material is efficiently supplied with liquid. The wicking action also assists in bringing liquid into contact with the upper cover layer to aid increase transpiration rates of the dressing.

The wound dressing layers of the electronics area and the absorbent layer can be covered by one continuous cover layer or backing layer 113. As used herein, the terms cover layer and/or backing layer can be used interchangeably to refer to the layer of material in the dressing configured to cover the underlying dressing layers and seal to the wound contact layer and/or the skin surrounding the wound. In some embodiments, the cover layer can include a moisture vapor permeable material that prevents liquid exudate removed from the wound and other liquids from passing through, while allowing gases through.

The cover layer 113 is preferably gas impermeable, but moisture vapor permeable, and can extend across the width of the wound dressing 100. The cover layer 113, which may for example be a polyurethane film (for example, Elastollan SP9109) having a pressure sensitive adhesive on one side, is impermeable to gas and this layer thus operates to cover the wound and to seal a wound cavity over which the wound dressing is placed. In this way an effective chamber is made between the cover layer 113 and a wound site where a negative pressure can be established. The cover layer 113 is preferably sealed to the wound contact layer 110 in a border region around the circumference of the dressing, ensuring that no air is drawn in through the border area, for example via adhesive or welding techniques. The cover layer 113 protects the wound from external bacterial contamination (bacterial barrier) and allows liquid from wound exudates to be transferred through the layer and evaporated from the film outer surface. The cover layer 113 preferably comprises two layers; a polyurethane film and an adhesive pattern spread onto the film. The polyurethane film is preferably moisture vapor permeable and may be manufactured from a material that has an increased water transmission rate when wet. In some embodiments, the moisture vapor permeability of the cover layer increases when the cover layer becomes wet. The moisture vapor permeability of the wet cover layer may be up to about ten times more than the moisture vapor permeability of the dry cover layer.

The electronics area 161 can include a source of negative pressure (such as a pump) and some or all other components of the TNP system, such as power source(s), sensor(s), connector(s), user interface component(s) (such as button(s), switch(es), speaker(s), screen(s), etc.) and the like, that can be integral with the wound dressing. For example, the electronics area 161 can include a button or switch 114 as shown in Figures 1A-1B. The button or switch 114 can be used for operating the pump (such as, turning the pump on/off).

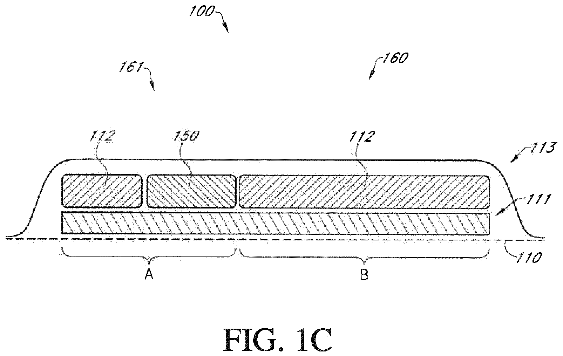

The absorbent area 160 can include an absorbent material 112 and can be positioned over the wound site. The electronics area 161 can be positioned away from the wound site, such as by being located off to the side from the absorbent area 160. The electronics area 161 can be positioned adjacent to and in fluid communication with the absorbent area 160 as shown in Figures 1A-1C. In some embodiments, each of the electronics area 161 and absorbent area 160 may be rectangular in shape and positioned adjacent to one another. In Figure 1C, the electronics area 161 is noted as area "A" and the absorbent area 160 is noted as area "B". In some embodiments, as illustrated in Figure 1C, electronic components 150 can be positioned within a recess or cut out of the absorbent material 112 but off to the side of the absorbent area. As shown in the cross sectional view of the wound dressing layers in Figure 1C, the absorbent material 112 can be positioned on both sides of the electronic components 150.

In some embodiments, additional layers of dressing material can be included in the electronics area 161, the absorbent area 160, or both areas. In some embodiments, the dressing can comprise one or more transmission or spacer layers and/or one or more absorbent layer positioned above the wound contact layer 110 and below the cover layer 113 of the dressing.

In some embodiments, the electronics area 161 of the dressing can comprise electronic components 150. In some embodiments, the electronics area 161 of the dressing can comprise one or more layers of transmission or spacer material and/or absorbent material and electronic components 150 can be embedded within the one or more layers of transmission or spacer material and/or absorbent material. The layers of transmission or absorbent material can have recesses or cut outs to embed the electronic components 150 within whilst providing structure to prevent collapse. The electronic components 150 can include a pump, power source, controller, and/or an electronics package.

A pump exhaust can be provided to exhaust air from the pump to the outside of the dressing, as described herein. The pump exhaust can be in communication with the electronics area 161 and the outside of the dressing.

As used herein the upper layer, top layer, or layer above refers to a layer furthest from the surface of the skin or wound while the dressing is in use and positioned over the wound. Accordingly, the lower surface, lower layer, bottom layer, or layer below refers to the layer that is closest to the surface of the skin or wound while the dressing is in use and positioned over the wound. Additionally, the layers can have a proximal wound-facing face referring to a side or face of the layer closest to the skin or wound and a distal face referring to a side or face of the layer furthest from the skin or wound.

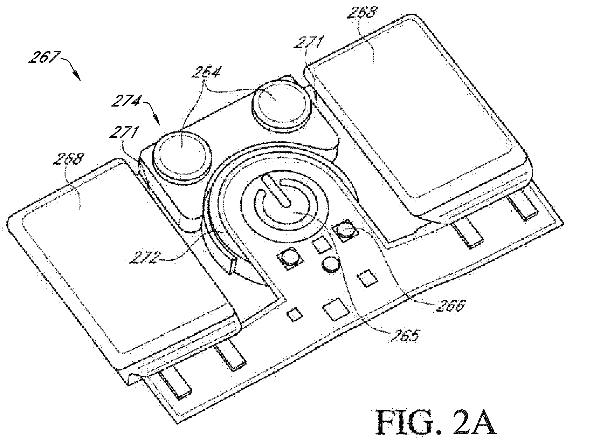

Figure 1A-1C illustrates a wound dressing apparatus incorporating the pump and/or other electronic components within the wound dressing and offset from the absorbent layer. In some embodiments, as shown in Figure 1C, the absorbent area 160 comprises a transmission layer 111 positioned above the wound contact layer 110. An absorbent layer 112 can be provided above the transmission layer 111. In some embodiments, the electronics area 161 can include an electronics unit (shown in Figures 2A-2C). In some embodiments, the electronics unit is provided directly over the wound contact layer. In other embodiments, the electronics unit can be placed above a layer of wicking material, absorbent material, or transmission material that sits above the wound contact layer 110 of the dressing. For example, as shown in Figure 1C, the electronics unit 150 may be positioned over the transmission layer 111. In some embodiments, the transmission layer 111 can be a single layer of material extending below the electronics unit 150 and the absorbent material 112. Thus, in some embodiments, the transmission layer 111 extends continuously through the absorbent area 160 and the electronics area 161. In alternative embodiments, the transmission layer below the electronics unit can be a different transmission layer than the transmission layer below the absorbent material 112. The transmission layer 111, absorbent material 112, and electronics unit 150 can be covered with a cover layer 113 that seals to a perimeter of the wound contact layer 110 as shown in Figures 1A-1C.

The electronics area 161 can include an electronics unit 150 positioned below the cover layer 113 of the dressing. In some embodiments, the electronics unit can be surrounded by a material to enclose or encapsulate a negative pressure source and electronics components by surrounding the electronics. In some embodiments, this material can be a casing. In some embodiments, the electronics unit can be encapsulated or surrounded by a protective coating, for example, a hydrophobic coating as described herein. The electronics unit can be in contact with the dressing layers in the absorbent area 160 and covered by the cover layer 113. As used herein, the electronics unit includes a lower or wound facing surface that is closest to the wound and an opposite, upper surface, furthest from the wound when the wound dressing is placed over a wound.

Figure 1C illustrates an embodiment of a wound dressing incorporating an electronics unit 150 within the dressing. In some embodiments, the electronics sub assembly or electronics unit 150 can be embedded in an aperture or hole in an absorbent layer 112 towards one end of the dressing, as depicted in Figure 1C.

In some embodiments, the absorbent components and electronics components can be overlapping but offset. For example, a portion of the electronics area can overlap the absorbent area, for example overlapping the superabsorber layer, but the electronics area is not completely over the absorbent area. Therefore, a portion of the electronics area can be offset from the absorbent area. The dressing layer and electronic components can be enclosed in a wound contact layer 110 positioned below the lower most layer and a cover layer 113 positioned above the absorbent layer 112 and electronics 150. The wound contact layer 110 and cover layer 113 can be sealed at a perimeter enclosing the dressing components. In some embodiments, the cover layer can be in direct physical contact with the absorbent material, and/or the electronics unit. In some embodiments, the cover layer can be sealed to a portion of the electronics unit and/or casing, for example, in areas where holes or apertures are used to accommodate the electronic components (such as, a switch and/or exhaust).

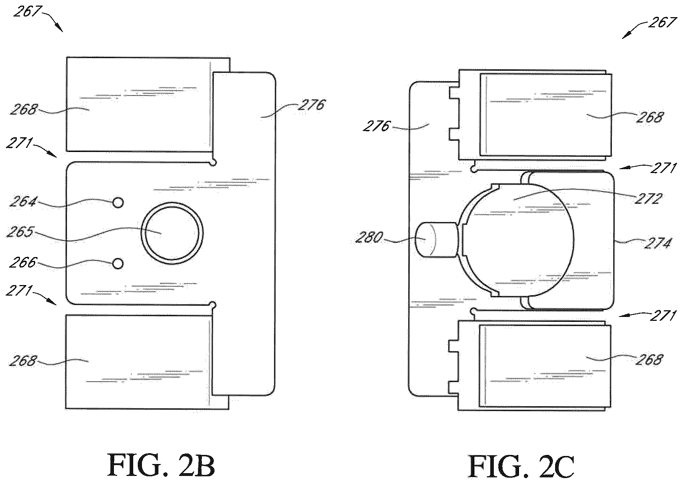

Figures 2A-2C illustrate embodiments of an electronics unit 267 that can be incorporated into a wound dressing. Figure 2A illustrates a perspective top view of the electronics unit (shown without an electronics casing or other dressing material). Figure 2B illustrates a top view of the electronics unit. Figure 2C illustrates a bottom or wound facing surface of the electronics unit. The electronics unit 267 can include a pump 272 and one or more batteries 268. The electronics unit 267 can include a circuit board 276 configured to be in electrical communication with the pump 272 and/or batteries 268. The circuit board 276 can be flexible or substantially flexible.

As illustrated in Figures 2A-2B, the electronics unit 267 can include single button or switch 265 on the upper surface of the unit. The single button or switch 265 can be used as an on/off button or switch to stop and start operation of the pump and/or electronic components. The switch 265 can be a dome type switch configured to sit on the top of the pump. Because the switch is situated within the dressing the cover layer can be easily sealed around or over the switch. In some embodiments, the cover layer can have an opening or hole positioned above the switch. The cover layer can be sealed to the outer perimeter of the switch 265 to maintain negative pressure under the wound cover. The switch can be placed on any surface of the electronics unit and can be in electrical connection with the pump.

The electronics unit 267 can also include one or more vents or exhaust apertures 264 on the circuit board 276 for expelling the air exhausted from the pump. As shown in Figure 2C, a pump outlet exhaust mechanism 274 (sometimes referred to as pump exhaust mechanism or pump outlet mechanism) can be attached to the outlet of the pump 272. The one or more vents or exhaust apertures 264 can be in fluid communication with a pump exhaust mechanism 274 positioned at the outlet of the pump and extending to the lower surface of the circuit board 276. In some embodiments, one or more of the vents 264 on the circuit board 276 can provide communication with the top surface of the dressing and allow the pump exhaust to be vented from the electronics unit. In some embodiments, the exhaust mechanism 274 can be attached to the outlet end of the pump and can extend out from the pump at a 90-degree angle from the pump orientation to communicate with the bottom surface of the circuit board 276. In some embodiments, the exhaust mechanism 274 can include an antibacterial membrane and/or a non-return valve. In some embodiments, the one or more vents 264 can include an antibacterial membrane and/or a non-return valve. The exhausted air from the pump can pass through the pump outlet and exhaust mechanism 274. In some embodiments, the cover layer 113 can include apertures or holes positioned above the one or more vents 264 and/or membrane. The cover layer 113 can be sealed to the outer perimeter of the one or more vents 264 to maintain negative pressure under the wound cover 113. In some embodiments, the exhausted air can be exhausted through the gas permeable material or moisture vapor permeable material of the cover layer. In some embodiments, the cover layer does not need to contain apertures or holes over the exhaust and the exhausted air is expelled through the cover layer. In some embodiments, the pump outlet mechanism 274 can be a custom part formed to fit around the pump as shown in Figures 2A and 2C. The electronic unit 267 can include a pump inlet protection mechanism 280 as shown in Figure 2C positioned on the portion of the electronic unit closest to the absorbent area and aligned with the inlet of the pump 272. The pump inlet protection mechanism 280 is positioned between the pump inlet and the absorbent area or absorbent layer of the dressing. As described herein, the pump inlet protection mechanism 280 can include hydrophobic material to prevent fluid from entering the pump 272. The pump inlet protection mechanism 280 (or any of the inlet protection mechanisms disclosed herein) can include a filter.

In some embodiments, the upper surface of the electronics unit can include one or more indicators 266 for indicating a condition of the pump and/or level of pressure within the dressing. The indicators can be small LED lights or other light source that are visible through the dressing components or through holes in the dressing components above the indicators. The indicators can be green, yellow, red, orange, or any other color. For example, there can be two lights, one green light and one orange light. The green light can indicate the device is working properly and the orange light can indicate that there is some issue with the pump (such as, leak, saturation level of the dressing, blockage downstream of the pump, exhaust blockage, low battery, or the like).

The electronics unit 267 can include a pump 272 and one or more batteries 268 or other power source to power the pump 272 and other electronics. The pump can operate at about 27 volts or about 30 volts. The two batteries can allow for a more efficient voltage increase (6 volts to 30 volts) than would be possible with a single battery.

The batteries 268 can be in electrical communication with the circuit board 276. In some embodiments, one or more battery connections are connected to a surface of the circuit board 276. In some embodiments, the circuit board 276 can have other electronics incorporated within. For example, the circuit board 276 may support various sensors including, but not limited to, one or more pressure sensors, temperature sensors, optic sensors and/or cameras, and/or saturation indicators.

In such embodiments, the components of the electronics unit 267 may include a protective coating to protect the electronics from the fluid within the dressing. The coating can provide a means of fluid separation between the electronics unit 267 and the absorbent materials of the dressing. The coating can be a hydrophobic coating including, but not limited to, a silicone coating or polyurethane coating. In some embodiments, the electronics unit 267 can be encapsulated in a protective housing or enclosure as described in more detail herein. The pump inlet component or pump inlet protection mechanism can be used to protect the pump from fluid on the inlet and the pump outlet mechanism can include a non-return valve that protects fluid from entering the outlet as described in more detail with reference to PCT International Application No. PCT/EP2017/055225, filed March 6, 2017, titled WOUND TREATMENT APPARATUSES AND METHODS WITH NEGATIVE PRESSURE SOURCE INTEGRATED INTO WOUND DRESSING and PCT International Application No. PCT/EP2017/059883, filed April 26, 2017, titled WOUND DRESSINGS AND METHODS OF USE WITH INTEGRATED NEGATIVE PRESSURE SOURCE HAVING A FLUID INGRESS INHIBITION COMPONENT, which are hereby incorporated by reference in their entireties. The pump inlet component or pump inlet protection mechanism can be a component that inhibits fluid ingress. The pump inlet component or pump inlet protection mechanism can allow gas (for example, air) but inhibit liquid (for example, wound exudate) from passing through. The pump inlet component or pump inlet protection mechanism can be a porous structure that provides a plurality of flow paths between an interior of the wound dressing and the pump. The plurality of flow paths can inhibit occlusion (for example, from wound exudate) of the pump. In some embodiments, the component can be made of or coated with a hydrophobic material that repels wound exudate, thereby inhibiting the ingress of fluid into the component and ultimately the pump.

The electronics unit 267 includes one or more slits, grooves or recesses 271 in the unit between the pump and the two batteries. The slits, grooves or recesses 271 can allow for the electronics unit 267 to be flexible and conform to the shape of the wound. The unit 267 can have two parallel slits, grooves or recesses 271 forming three segments of the electronics unit 267. The slits, grooves or recesses 271 of the unit 267 create hinge points or gaps that allows for flexibility of the electronics unit at that hinge point. The one or more pump vents 264, switch 265, and indicator 266 are shown on the top surface of the electronics unit 267. As illustrated, one embodiment of the electronics unit 267 has two hinge points to separate the unit into three regions or panels, for example one to contain one battery, one to contain the pump, and one to contain another battery. In some embodiments, the slits, grooves or recesses may extend parallel with a longitudinal axis of the dressing that extends along the length of the dressing through the electronics area of the dressing through the absorbent area of the dressing.

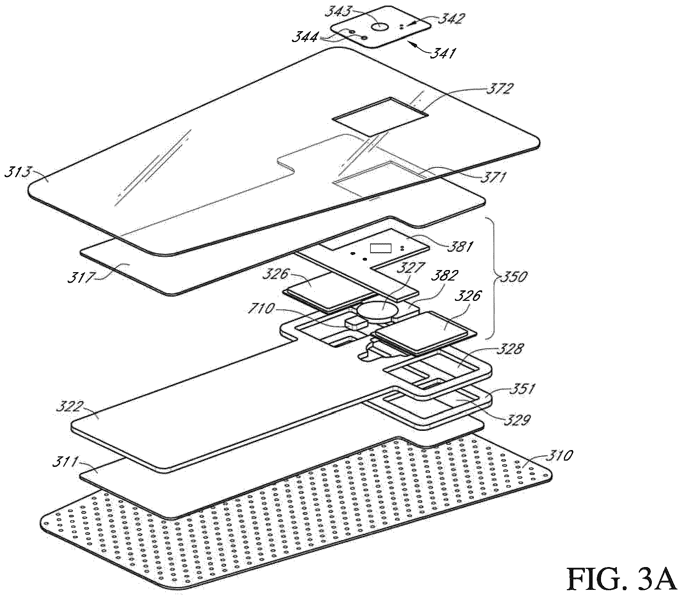

Figure 3A illustrates an embodiment of wound dressing layers incorporating the electronic components within the wound dressing. Figure 3A illustrates a wound dressing with a wound contact layer 310 configured to contact the wound. The wound contact layer 310 can be a similar material and have a similar function as the wound contact layer described with reference to Figures 1A-1C. A transmission layer or spacer layer 311 is provided over the wound contact layer. The transmission layer or spacer layer 311 can be a similar material and have a similar function as the transmission layer or spacer layer described with reference to Figures 1A-1C. The transmission layer 311 can assist in transmitting and distributing negative pressure over the wound site.

A first layer of apertured absorbent material 351 can be provided over the transmission layer 311. The first apertured absorbent layer 351 can include one or more apertures 329. In some embodiments, the apertures 329 can be sized and shaped to fit the electronics unit 350 therein. The first apertured absorbent layer 351 can be sized and shaped to the size of the electronics area and does not extend into the absorbent area. In some embodiments, the apertures 329 can be shaped and sized to fit the individual components of the electronics unit 350.

A second apertured absorbent layer 322 can be provided over the first absorbent layer 351. **In** some embodiments, the second absorbent layer 322 includes one or more apertures 328. The second absorbent layer 322 can be sized and shaped to the size of the electronics area and the absorbent area. In some embodiments, the apertures 328 can be shaped and sized to fit the individual components of the electronics unit 350. The first and second absorbent layers 351 and 322 can be a similar material and have a similar function as the absorbent layer described with reference to Figures 1A-1C.

An electronics unit 350 can be positioned in the apertures 328 and 329 of the first and second absorbent material 351 and 322. The electronics unit 350 can be similar to the electronics unit described with reference to Figures 2A-2C. The electronics unit 350 can include a pump 327, power source 326, and a printed circuit board 381. In some embodiments, the pump 327 can include a pump inlet mechanism 710 and an outlet mechanism 382. In some embodiments, the printed circuit board 381 can include electronics including but not limited to a switch, sensors, and LEDs as described herein. In some embodiments, the circuit board 381 can include one or more hole to be positioned over one or more vents (not shown) in the outlet mechanism 382 as described in more detail herein.

An overlay layer 317 can be provided over the electronics components 350 and absorbent layer 322. In some embodiments, the overlay layer 317 can be one or more layers of absorbent and/or transmission material as described herein. In some embodiments, the overlay layer 317 can comprise a conformable material overlaying and overbordering the perimeter of the lower layers of transmission and absorbent materials. In some embodiments, the overlay layer 317 can soften the edges of the wound dressing layers by decreasing the profile around the edges of the dressing layers. The overlay layer 317 can protect the cover layer from being punctured by the lower layers when the cover layer is sealed over the dressing layers below. The overlay layer 317 can include an aperture 371 to allow access to at least a portion of the electronics unit 350 positioned below.

A cover layer or backing layer 313 can be positioned over the overlay layer 317. The cover layer or backing layer 313 can be a similar material and have a similar function as the cover layer or backing layer described with reference to Figures 1A-1C. In some embodiments, when the overlay layer 317 is not used, the cover layer or backing layer 313 can be provided above absorbent layers 322, and/or electronic components 350. The cover layer 313 can form a seal to the wound contact layer 310 at a perimeter region enclosing the overlay layer 317, absorbent layers 322 and 351, electronic components 350, and the transmission layer 311. In some embodiments, the cover layer 313 can be a flexible sheet of material that forms and molds around the dressing components when they are applied to the wound. In other embodiments, the cover layer 313 can be a material that is preformed or premolded to fit around the dressing components as shown in Figure 3A. As used herein, the terms cover layer and backing layer can be used interchangeably to refer to the layer of material in the dressing configured to cover the layers of the wound dressing.

In some embodiments, the cover layer or backing layer 313 can include an aperture 372. The aperture 372 can be positioned over at least a portion of the aperture 371 in the overlay layer 317 to allow access to at least a portion of the electronics unit 350 positioned below. In some embodiments, the apertures 371 and 372 can allow access to the switch and/or vents of the pump exhaust.

A label 341 can be provided over the apertures 371 and 372 and positioned over the exposed portion of the electronic components 350. The label can include one or more apertures or vents 342, indicator portions 344, and/or switch cover 343. The indicator portions 344 can include holes or transparent regions 344 for positioning over the one or more indicators or LEDs on the printed circuit board 381 below the label 341. The holes or transparent regions 344 can allow for the indicators or LEDs to be visible through the label 341. In some embodiments, the switch cover 343 can include a dome shaped cover positioned over the switch on the printed circuit board 381. In some embodiments, the label 341 can include embossed features for the switch cover 343. In some embodiments, the embossed features of the switch cover 343 can prevent accidental activation or deactivation of the device. In some embodiments, the switch or switch cover 343 can include a tab on the switch to prevent accidental activation or deactivation. The one or more vents 342 of the label can allow exhaust from the pump outlet mechanism to pass through the label and exit the wound dressing to be exhausted to the atmosphere.

In some embodiments, the label can be positioned on top of the cover layer or backing layer 313. The label can seal to the cover layer to form a seal over the wound. In other embodiments, the label 341 can be positioned above the overlay layer 371 and below the cover layer or backing layer 313. In such embodiments, the cover layer 313 can have one or more apertures over one or more components of the label 341. For example, the cover layer 313 can have apertures over the one or more vents 342, indicator portions 344, and/or switch cover 343.

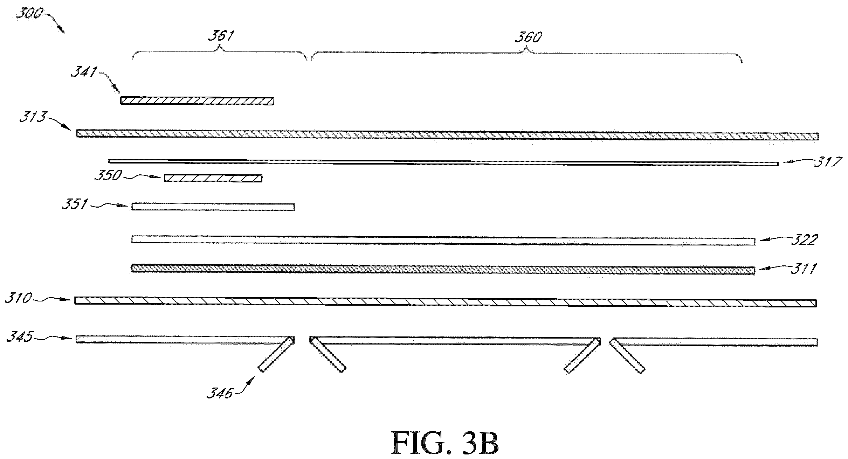

Figure 3B illustrates a cross sectional layout of the material layers of the wound dressing incorporating an electronics assembly within the dressing. The dressing 300 included multiple material layers and an electronics assembly 350. The wound dressing 300 can include an electronics area 361 including the electronics and an absorbent area or dressing area 360 that is intended to be applied to the wound as described with reference to Figures 1A-1C.

As described herein, the one or more material layers can extend into both the electronics area 361 and the dressing area 360. The dressing 300 can include a wound contact layer 310, transmission layer 311, absorbent layers 322 and 351, an overlay layer 317, and a cover or backing layer 313 as illustrated in Figure 3B. The absorbent layers 322 and 351 can include recesses or cutouts to receive the components of the electronics assembly 350 as described herein. In some embodiments, as illustrated in Figure 3B the small apertured absorbent layer 351 can be positioned on top of the large apertured absorbent layer 322. In other embodiments, as illustrated in Figure 3A the small apertured absorbent layer 351 can be positioned on below of the large apertured absorbent layer 322.

In some embodiments, the overlay layer 317 and/or the cover layer 313 can include a cut out or aperture positioned over the switch and/or indicators of the electronics assembly 350. A label or covering 341 can be positioned over at least a portion of the electronics assembly 350 and any cutouts in the overlay layer 317 and/or the cover layer 313. The label or covering 341 can be similar to the label or covering 341 as described previously with reference to Figure 3A.

Before use, the dressing can include a delivery layer 345 adhered to the bottom surface of the wound contact layer. The delivery layer 345 can cover adhesive or apertures on the bottom surface of the wound contact layer 310. In some embodiments, the delivery layer 345 can provided support for the dressing and can assist in sterile and appropriate placement of the dressing over the wound and skin of the patient. The delivery layer 345 can include handles 346 that can be used by the user to separate the delivery layer 345 from the wound contact layer 310 before applying the dressing 300 to a wound and skin of a patient.

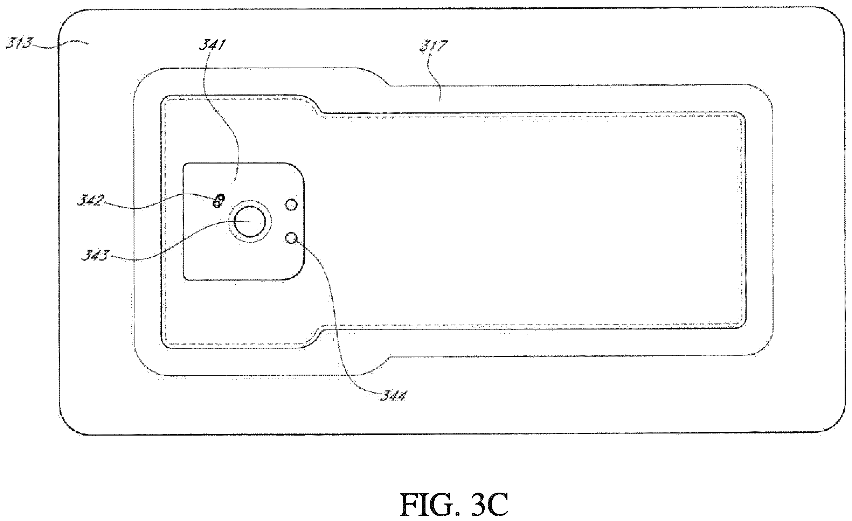

Figure 3C illustrates a top view of an embodiment of the wound dressing incorporating an electronic assembly within the dressing.

Figure 3C shows a cover layer 313 and electronics covering 341 covering the overlay layer 317 and underlying dressing and electronics components. The cover layer 313 can seal to the wound contact layer 310 at a perimeter region of the wound contact layer 310. In some embodiments, the label or electronics covering 341 can be positioned over the cover layer 313. In other embodiments, the cover layer 313 can seal over the electronics covering 341. In some embodiments, the cover layer 313 can include one or more holes in the cover layer 313 positioned over the switch and/or pump outlet vent(s). In some embodiments, the cover layer 313 can include a single hole that is positioned over the switch cover 343, visual indicators 344, and/or pump outlet vent(s) 342 in the covering or label 341 as shown in Figure 3C. In some embodiments, the label can include embossed features for the switch cover 343. In some embodiments, the embossed features of the switch cover 343 can prevent accidental activation or deactivation of the device. In some embodiments, the switch or switch cover 343 can include a tab on the switch to prevent accidental activation or deactivation.

The visual indicators 344 can provide an indication of operation of the negative pressure source and/or an indication of the level of negative pressure that is applied to the wound. In some embodiments, the visual indicators can include one or more light sources or LEDs. In some embodiments, the visual indicator light sources an illuminate to indicate a condition or change of condition. In some embodiments, the light source can illuminate in a particular sequence and/or color that indicates a condition. For example, in some embodiments, the light source can flash to notify the user that the device is operating properly. In some embodiments, the light source can automatically flash periodically and/or the light source can be activated by the switch or other button to light up and indicate a condition.

In some embodiments, the switch can be pressed and/or held down to power the dressing and electronics on and off. In some embodiments, once the switch is activated and the pump and associated colored LED, for example, green colored LED, can be used to confirm the dressing and integrated negative pressure source are operational. In some embodiments, during operation of the pump and dressing, the pump and dressing can enter the fault state indicated by a colored LED, for example, orange colored LED.

### Electronic Assembly

The wound dressing described herein can utilize the embedded electronic assembly to generate negative pressure under the dressing. However, it can be important to protect the assembly from wound exudate or other bodily fluids that would corrode the electronics. It can also be important to protect the patient from the electric and electronic components. The electronics assembly can incorporate a pump that pull air from the dressing and exhaust to the environment in order to produce the required negative pressure differential. Therefore, it can be difficult to protect the electronics assembly and allow fluid communication between the electronic assembly and the dressing and environment surrounding the dressing. For example, complete encapsulation or potting of the assembly could prevent the movement of air from the dressing and atmosphere to the pump. In some embodiments, described previously herein, the electronic components of the electronics assembly can be protected from the environment by partial encapsulation, potting, and/or a conformable coating. In some embodiments, potting of electronic components can include a process of filling a complete electronic assembly with a solid or gelatinous compound for resistance to shock and vibration, exclusion of moisture, and/or exclusion of corrosive agents.

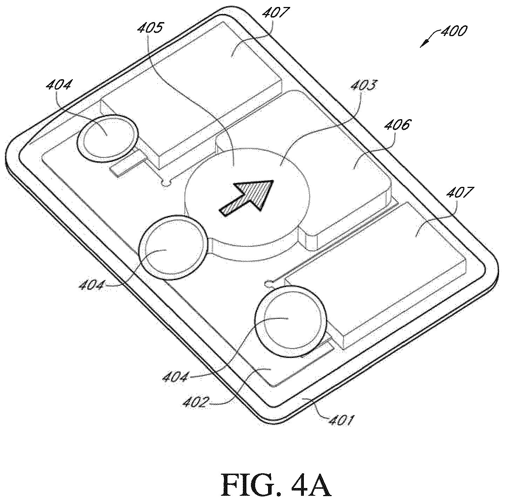

An electronics assembly can be used that includes an electronics unit positioned within an enclosure or housing, as illustrated in Figure 4A, to be incorporated into a wound dressing. The electronics unit enclosed in the housing can be similar to the electronics unit described with reference to Figures 2A-2C but the electronics unit can be positioned within an enclosure or housing. The housing with the electronics unit enclosed within can be placed in the dressing. Figures 4A-4B illustrates an embodiment of an electronics assembly 400 enclosing an electronics unit 403 within a housing.

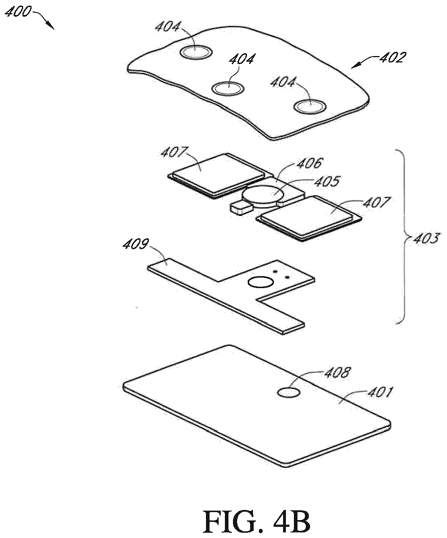

As illustrated in Figures 4A and 4B, the housing of the electronics assembly 400 can include a plate 401 and flexible film 402 enclosing the electronics unit 403 within. The electronics unit 403 can include a pump 405, inlet protection mechanism 410 (shown in Figure 4B), pump exhaust mechanism 406, power source 407, and a circuit board 409. The circuit board 409 can be flexible or substantially flexible. As is illustrated, the ump exhaust mechanism 406 can be an enclosure, such as a chamber. In some embodiments, the electronics unit 403 and pump 405 can be used without the inlet protection mechanism 410. The flexible film 402 can be attached to the plate 401 by welding (heat welding) or adhesive bonding to form a fluid tight seal and enclosure around the electronic components. In some embodiments, the flexible film 402 can be attached to the plate at a perimeter of the plate by heat welding, adhesive bonding, ultrasonic welding, RF welding, or any other attachment or bonding technique.

The flexible film 402 can be a flexible plastic polymeric film. In some embodiments, the flexible film 402 can be formed from any material flexible polymeric film or any flexible material that confirms around the electronics. The flexible film can maintain conformability and flexibility while protecting and insulating the components within. In some embodiments, the flexible film 402 can be formed from a flexible or stretchable material, such as one or more of polyurethane, thermoplastic polyurethane (TPU), silicone, polycarbonate, polyethylene, methylated polyethylene, polyimide, polyamide, polyester, polyethelene tetraphthalate (PET), polybutalene tetreaphthalate (PBT), polyethylene naphthalate (PEN), polyetherimide (PEI), along with various fluropolymers (FEP) and copolymers, or another suitable material. In some embodiments, the flexible film 402 can be formed from polyurethane.

The plate 401 can be a plastic polymer plate. In some embodiments, the plate can be a flexible material to allow conformability to movement or flexing of the dressing when it is applied to a wound. In some embodiments, the plate can be integrated with the components of the label described with reference to Figures 3A-3C. In other embodiments, the label can be a separate component attached to the top surface of the plate 401.

The flexible film 402 and plate 401 can be waterproof to protect the electronics unit 403 from fluid within the dressing. In some embodiments, the flexible film 402 can be sized appropriately so as not to limit the flexibility of the assembly. In some embodiments, depending on the properties of the film 402, the electronics assembly 400 can be thermoformed or vacuum formed to assist in the function of maintaining the flexibility of the assembly. In some embodiments, the electronics unit 403 can be bonded or adhered to the plate 401 within the housing such that the electronics unit 403 cannot move within.

In some embodiments, the housing can include one or more windows 404. The windows 404 can be a porous film or membrane that can allow gas to pass through. The windows 404 can be a hydrophobic film or membrane. In some embodiments, the hydrophobic nature of the window 404 can repel wound fluids and prevent the leak of fluids into the electronics assembly 400. In some embodiments, the windows 404 can include a bacterial filter. In some embodiments, the windows 404 can have the porosity that enables them to act as a bacterial filter and preventing bacterial release from the body fluids into the environment. The windows 404 can also prevent the ingress of bacteria from the environment to the wound site.

The electronics assembly 400 can have more than one window 404 or a larger window 404 to provide a sufficiently large area for air movement therethrough, thus minimizing the pressure drop across the membrane and hence the power consumption of the system in achieving the pressure differential. In some embodiments, as illustrated in Figures 4A-4B, the electronics assembly 400 can include several windows with a small area. In other embodiments, the electronics assembly can include a window with a single large area.

The electronics assembly 400 illustrated in Figures 4A-4B can be incorporated within the wound dressing such that, once the dressing is applied to the body of the patient, air from within the dressing can pass through the windows 404 to be pumped out in the direction shown by the arrow on the pump 405. The exhausted air from the pump can pass out of the pump assembly through the pump exhaust mechanism 406 and be exhausted or vented from the housing of the electronics assembly 400 through an aperture or vent 408 in the plate 401. Although a single vent 408 is illustrated, in some cases multiple vents can be provided. In some embodiments, the circuit board 409 can be positioned between the exhaust mechanism 406 and the plate 401. The circuit board 409 can also include an aperture or vent aligned with the exhaust hole in the exhaust mechanism as described with reference to Figures 2A-2C. The one or more vents or apertures in the exhaust mechanism 406, circuit board 409, and plate 401 can be aligned and sealed to each other. This seal can ensure the pump exhaust is exhausted from the electronics assembly 400 through the at least one vent 408 in the plate 401. In other embodiments, the exhaust mechanism 406 of the electronics unit 403 can be positioned on and bonded directly to the plate 401 with an air tight seal.

The top side of the plate 401 (not shown in Figures 4A-4B) can include a label similar to the label described with reference to Figures 3A-3C. In other embodiments, the top side of the plate 401 can integrate the components of the label described with reference to Figure 3A-3C within the plate 401. In such embodiments, a separate label is not needed. For example, in addition to the at least one vent 408, the plate 401 can include the indicator portions and/or a switch cover described previously herein.

In some embodiments, the electronics assembly 400 can be embedded within the wound dressing in the same manner as the electronics unit described with reference to Figures 3A-3C. The dressing can have one or more absorbent layers within the dressing and the absorbent layers can have a single aperture or recess for receiving the electronics assembly within. In some embodiments, the electronics assembly can be positioned below the overlay layer similar to the electronics unit described with reference to Figures 3A-3C. In such embodiments, the overlay layer would include an aperture to allow access to at least a portion of the top surface of the plate 401.

When the electronics assembly 400 is positioned within the dressing it can be positioned below the wound cover and the overlay layer similar to the electronics unit described with reference to Figures 3A-3C. In other embodiments, an overlay layer is not used and the electronics assembly 400 is positioned directly below the cover layer or backing layer.

The cover layer or backing layer can include an aperture exposing a portion of, most of, or all of the top surface of the plate 401. The aperture in the cover layer can be positioned over at least a portion of the plate 401 to allow access to at least a portion of the plate 401 positioned below the cover layer. In some embodiments, the cover layer can have a plurality of apertures over one or more components of the label or top surface of the plate 401. For example, the cover layer can have one or more apertures or vents over the one or more vents, indicator portions, and/or switch cover. In other embodiments, the cover layer can have a single aperture over the one or more components of the label or top surface of the plate 401, including but not limited to the vents, indicator portions, and/or switch cover.

When a separate label is used, it can be applied to the dressing and exposed portion of the plate 401 as described with reference to Figures 3A-3C, above or below the cover layer.

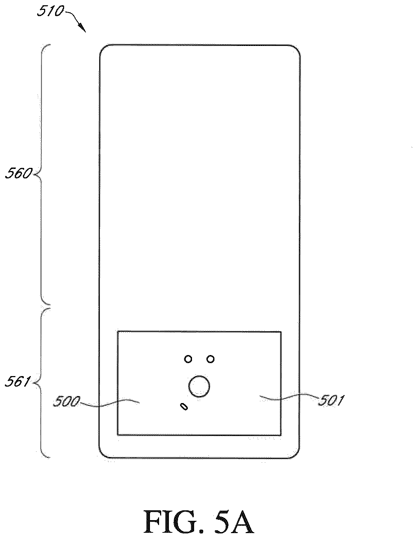

Figures 5A-5B illustrate embodiments of the electronics assembly 500 positioned within an aperture in wound dressing 510 layers. As illustrated in Figures 5A-5B, the dressing 510 can include an absorbent area 560 and an electronics area 561 similar to the corresponding components described with reference to Figures 1A-1C and 3A-3C. The dressing can have one or more dressing layers similar to the layers described with reference to Figures 1A-1C and 3A-3C. The dressing layers can have a single aperture or recess for receiving the electronics assembly within.

The wound dressing 510 can be formed from a wound contact layer, a transmission layer, and one or more absorbent layers as shown in Figures 1A-C and 3A-3C. The one or more absorbent layers can have a single aperture to receive the electronics assembly 500. The transmission layer and one or more absorbent materials can be covered with a cover layer 513 that seals to a perimeter of the wound contact layer as described with reference to Figures 1A-C. As illustrated in Figures 5A-5B, the overlay layer is not used. The aperture in the one or more absorbent layers can be aligned with the aperture 520 in the cover layer 513.

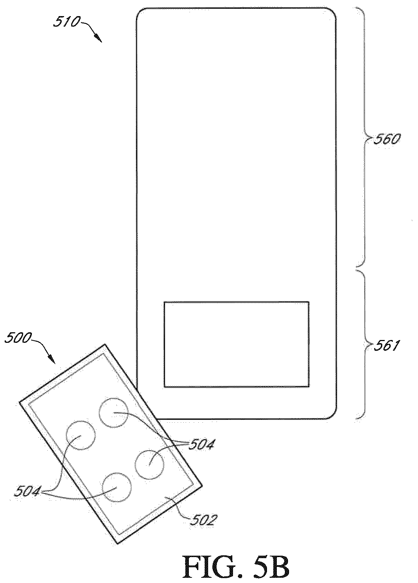

Figure 5A illustrates a top view of the electronics assembly 500 positioned in an electronics area 561 of the dressing 510. Figure 5A illustrates a cover layer 513 of the dressing 510 with an electronics assembly 500 positioned in a recess in the dressing. The other layers of the wound dressing below the cover layer are not shown. The electronics assembly 500 can be similar to the electronics assembly described with reference to Figures 4A-4B. The electronics assembly 500 can include an electronics unit enclosed within a housing including a plate 501 and a flexible film 502. The plate 501 shown in Figure 5A can include the features of the label including the one or more vents 542, one or more indicator portions 544, and/or a button or switch 543. Figure 5B illustrates an embodiment of the electronics assembly 500 removed from the electronics area 561 of the dressing 510. The electronics assembly 500 is shown upside down with the windows facing up.

The electronics assembly can have a first side positioned on the wound facing side of the electronics assembly 500 when the dressing 510 is positioned over the wound. As illustrated, the flexible film 502 and windows 504 can form the first wound facing side of the electronics assembly 500 in contact with the dressing layer and facing the wound when the dressing is positioned over the wound. The electronics assembly 500 can have a second side opposite the first side. The plate 501 can form the second side of the electronics assembly and can be in contact with the environment when the dressing is positioned over the wound.

As illustrated in Figure 5B, the flexible film 502 can have windows 504. When the electronics assembly 500 is positioned on or in the wound dressing as shown in Figure 5A, the windows 504 are in fluid communication with the layers within the wound dressing allowing the electronics assembly to generate negative pressure under the dressing 510.

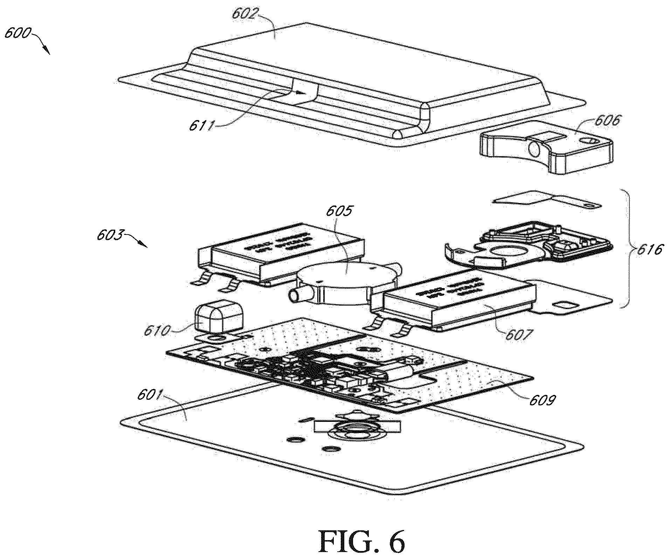

Figure 6 illustrates an embodiment of an electronics assembly 600 enclosing an electronics unit within a housing. As illustrated in Figure 6, the housing of the electronics assembly 600 can include a plate 601 and flexible film 602 enclosing the electronics unit 603 within. The electronics unit 603 can include a pump 605, inlet protection mechanism 610, pump exhaust mechanism 606, power source 607, and circuit board 609. The circuit board 609 can be flexible or substantially flexible.

The pump exhaust mechanism 606 can be similar to the pump exhaust mechanism 406. However, the pump exhaust mechanism 606 and the pump 605 can sit within an extended casing 616.

The flexible film 602 can be attached to the plate 601 by welding (heat welding) or adhesive bonding to form a fluid tight seal and enclosure around the electronic components. In some embodiments, the flexible film 602 can be attached to the plate at a perimeter of the plate by heat welding, adhesive bonding, ultrasonic welding, RF welding, or any other attachment or bonding technique.

The flexible film 602 can be a flexible plastic polymeric film. In some embodiments, the flexible film 602 can be formed from any material flexible polymeric film or any flexible material that confirms around the electronics. The flexible film can maintain conformability and flexibility while protecting and insulating the components within. In some embodiments, the flexible film 602 can be formed from a flexible or stretchable material, such as one or more of polyurethane, thermoplastic polyurethane (TPU), silicone, polycarbonate, polyethylene, methylated polyethylene, polyimide, polyamide, polyester, polyethelene tetraphthalate (PET), polybutalene tetreaphthalate (PBT), polyethylene naphthalate (PEN), polyetherimide (PEI), along with various fluropolymers (FEP) and copolymers, or another suitable material. In some embodiments, the flexible film 602 can be formed from polyurethane.

The plate 601 can be a plastic polymer plate. In some embodiments, the plate can be a flexible material to allow conformability to movement or flexing of the dressing when it is applied to a wound. In some embodiments, the plate can be integrated with the components of the label described with reference to Figures 3A-3C. In other embodiments, the label can be a separate component attached to the top surface of the plate 601. In some embodiments, the plate and/or label can have a larger surface area than the circuit board 609 and/or the electronics unit so that the flexible film 602 can seal to the outer perimeter of the plate and/or label around the circuit board 609 and/or the electronics unit

The flexible film 602 and plate 601 can be waterproof to protect the electronics unit 603 from fluid within the dressing. In some embodiments, the flexible film 602 can be sized appropriately so as not to limit the flexibility of the assembly. In some embodiments, depending on the properties of the film 602, the electronics assembly 600 can be thermoformed or vacuum formed to assist in the function of maintaining the flexibility of the assembly. In some embodiments, the electronics unit 603 can be bonded or adhered to the plate 601 within the housing such that the electronics unit 603 cannot move within.

In some embodiments, the flexible film 603 can include an aperture 611. The aperture 611 can allow the inlet protection mechanism 610 to be in fluid communication with the absorbent and/or transmission layers of the wound dressing. The perimeter of the aperture 611 of the flexible film 603 can be sealed or attached to the inlet protection mechanism 610 by welding (heat welding) or adhesive bonding to form a fluid tight seal and enclosure around the inlet protection mechanism 610 allowing the electronic components 603 to remain protected from fluid within the dressing. In some embodiments, the flexible film 602 can be attached to the inlet protection mechanism 610 at a perimeter of the inlet protection mechanism 610 by heat welding, adhesive bonding, ultrasonic welding, RF welding, or any other attachment or bonding technique. The inlet protection mechanism 610 can prevent wound exudate or liquids from the wound and collected in the absorbent area 660 of the wound dressing from entering the pump and/or electronic components of the electronics assembly 600.

The electronics assembly 600 illustrated in Figure 6 can be incorporated within the wound dressing such that, once the dressing is applied to the body of the patient, air from within the dressing can pass through the inlet protection mechanism 610 to be pumped out toward the pump exhaust mechanism 606 in communication with an aperture in the casing 616 and the circuit board 609 as described herein.

In some embodiments, the casing 616 can include one or more apertures or vents to allow the air exhausted from the pump exhaust mechanism 606 to pass through. The exhausted air from the pump can pass out of the pump assembly through the pump exhaust mechanism 606 and casing 616 and be exhausted or vented from the housing of the electronics assembly 600 through an aperture or vent in the plate 601. In some embodiments, the circuit board 609 can be positioned between the exhaust mechanism 606 and the plate 601. The circuit board 609 can also include one or more apertures or vents aligned with the one or more exhaust holes or vents in the exhaust mechanism as described with reference to Figures 2A-2C. The one or more vents in the exhaust mechanism 606, casing 616, circuit board 609, and plate 601 can be aligned and sealed to each other. This seal can ensure the pump exhaust is exhausted from the electronics assembly 600 through the at least one vent in the plate 601. In other embodiments, the exhaust mechanism 606 of the electronics unit 603 can be positioned on and bonded directly to the plate 601 with an air tight seal.

The top side of the plate 601 (not shown in Figure 6) can include a label similar to the label described with reference to Figures 3A-3C. In other embodiments, the top side of the plate 601 can integrate the components of the label described with reference to Figure 3A-3C within the plate 601. In such embodiments, a separate label is not needed. For example, in addition to the at least one vent, the plate 601 can include the indicator portions and/or a switch cover as described herein.

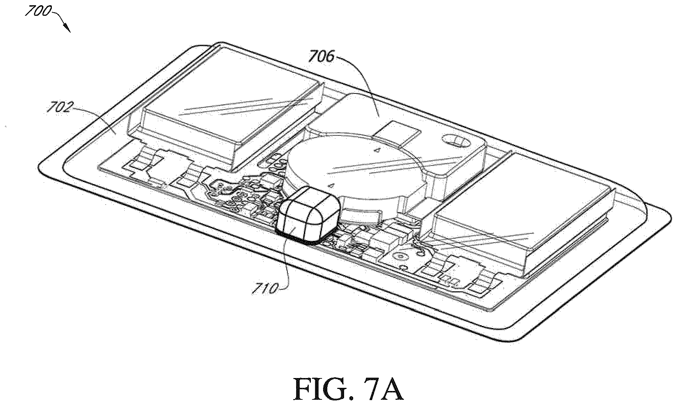

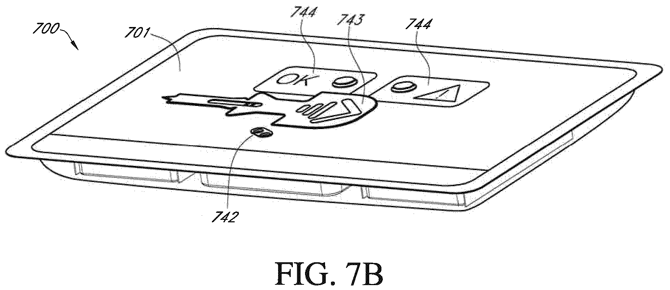

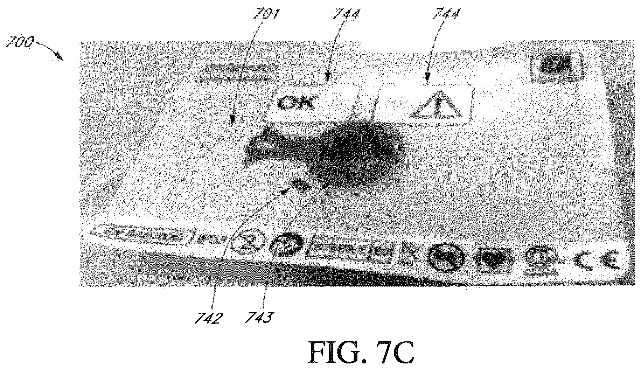

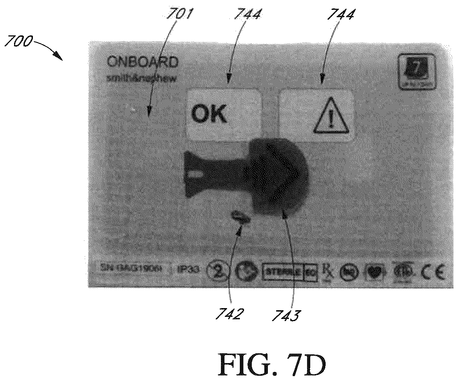

Figure 7A shows a lower wound facing surface of an electronics assembly 700. Figure 7 illustrat an electronics assembly including a pump inlet protection mechanism 710 sealed to the exterior of the flexible film 702, similar to the description with reference to Figure 6. Also shown is an exhaust mechanism 706, which can be similar to the exhaust mechanism 606.

Figures 7B-7D show an upper surface of the plate 701 of the electronics assembly 700. The upper surface of the plate can include an on/off switch or button cover 743, indicators 744, and/or one or more vent holes 742. The on/off switch cover or button 743, indicators 744, and/or vent(s) 742 can be similar to the switch cover or button and indictor portions described with reference to Figures 3A-3C, 4A-4B, and 5A-5B.

In some embodiments, as shown in Figures 7B-7D, the switch or button cover 743 can be positioned over the switch on a circuit board of the electronics components as described herein. In some embodiments, the plate can include embossed features for the switch cover 743. In some embodiments, the embossed features of the switch cover 743 can prevent accidental activation or deactivation of the device. In some embodiments, the switch or switch cover 743 can include a tab on the switch to prevent accidental activation or deactivation.

In some embodiments, as shown in Figures 7B-7D, the indicator portions can include visual symbols or words to indicate the condition of the wound dressing and electronics. For example, as shown in Figures 7B-7D, one indicator portion can read "OK". When the LED or light source associated with the "OK" indicator portion is illuminated the user is provided an indication that the dressing or electronics are functioning properly. An indicator portion can have a symbol, for example, a caution symbol similar to the symbol shown in Figures 7C-7D. When the LED or light source associated with the caution symbol on the indicator portion is illuminated the user is provided an indication that the dressing or electronics may not be functioning properly and/or there may be a leak.

The one or more vent holes 742 of the plate can allow exhaust from the pump outlet mechanism to pass through the plate and exit the wound dressing to be exhausted to the atmosphere.

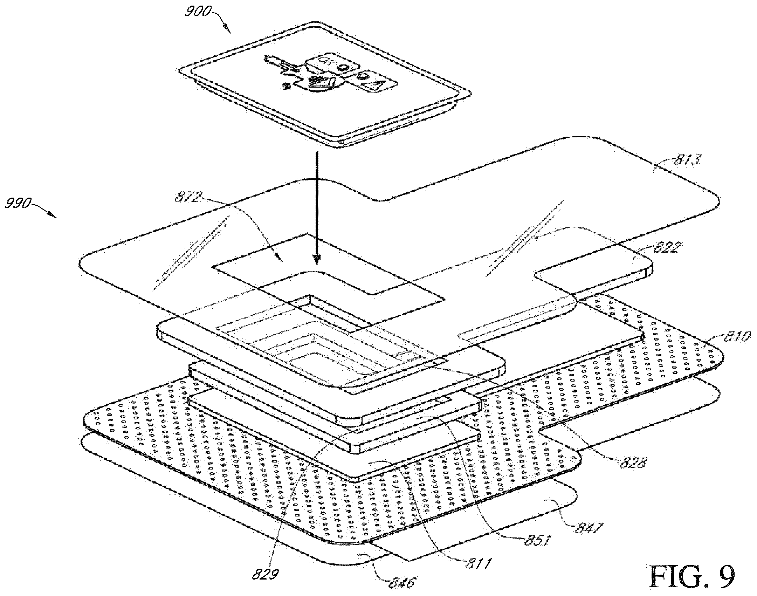

The electronics assembly 700 with the pump inlet protection mechanism 710 extending from and sealed to the film 702 can be positioned within the aperture 520 in the cover layer 513 and absorbent layer(s) (not shown) as shown in Figures 5A-5B and described in more detail herein. In some embodiments, the perimeter of the electronics assembly 700 can be sealed to a top surface of the outer perimeter of the aperture 520 in the cover layer 513 as shown in Figures 5A-5B and described in more detail with reference to Figure 9 herein. In some embodiments, the electronics assembly 700 is sealed to the cover layer 513 with a sealant gasket, adhesive, heat welding, adhesive bonding, ultrasonic welding, RF welding, or any other attachment or bonding technique. In some embodiments, the electronics assembly 700 can be permanently sealed to the cover layer 513 and could not be removed from the cover layer without destroying the dressing.

In some embodiments, the electronics assembly 700 can be utilized in a single dressing and disposed of with the dressing. In other embodiments, the electronics assembly 700 can be utilized in a series of dressings.

### Electronics Assembly Incorporated Within the Wound Dressing

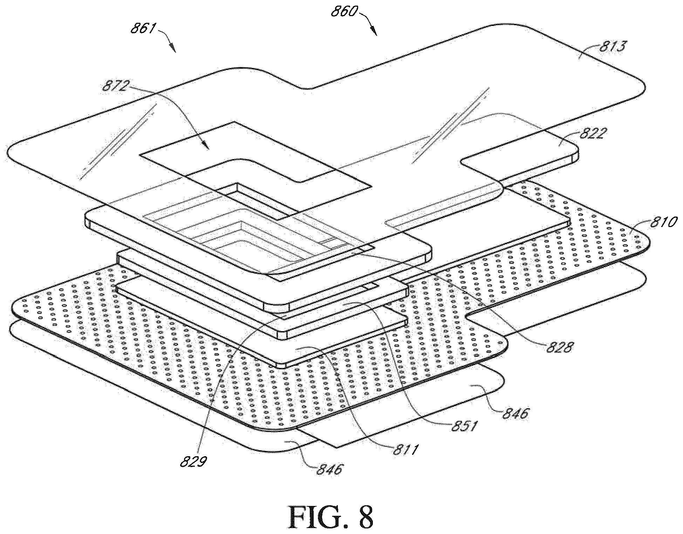

Figure 8 illustrates an embodiment of wound dressing layers for a wound dressing that can be used with the incorporates electronics components and/or electronics assembly described herein. The dressing layers and components of Figure 8 can be similar to the dressing layers and components described in Figure 3A. However, the wound dressing illustrated in Figure 8 can incorporate electronic components and negative pressure source enclosed within an electronics assembly similar to the electronics assembly 400, 500, 600, and 700 described with reference to Figures 4A-4B, Figures 5A-5B, Figure 6, and Figures 7A-7D. Figure 8 illustrates a wound dressing with a wound contact layer 810 configured to contact the wound. A transmission layer or spacer layer 811 is provided over the wound contact layer. The transmission layer 811 can assist in transmitting and distributing negative pressure over the wound site.

A first layer of apertured absorbent material 851 can be provided over the transmission layer 811. The first apertured absorbent layer 851 can include one or more apertures 829. In some embodiments, the aperture 829 can be sized and shaped to fit an electronics assembly and/or electronics unit therein. The first apertured absorbent layer 851 can be sized and shaped to the size of the electronics area 861 and does not extend into the absorbent area 860. In some embodiments, the aperture 829 can be shaped and sized to fit the electronics assembly formed from the plate and film described with reference to Figures 4-7.

A second apertured absorbent layer 822 can be provided over the first absorbent layer 851. **In** some embodiments, the second absorbent layer 822 includes one or more apertures 828. The second absorbent layer 822 can be sized and shaped to the size of the electronics area 861 and the absorbent area 860. In some embodiments, the aperture 828 can be shaped and sized to fit the electronics assembly formed from the plate and film described with reference to Figures 4-7.

A cover layer or backing layer 813 can be positioned over the absorbent material 822. The cover layer 813 can form a seal to the wound contact layer 810 at a perimeter region enclosing the absorbent layers 822 and 851 and the transmission layer 811. In some embodiments, the cover layer 813 can be a flexible sheet of material that forms and molds around the dressing components when they are applied to the wound. **In** other embodiments, the cover layer 813 can be a material that is preformed or premolded to fit around the dressing components. As used herein, the terms cover layer and backing layer can be used interchangeably to refer to the layer of material in the dressing configured to cover the layers of the wound dressing.

**In** some embodiments, the cover layer or backing layer 813 can include an aperture 872. The aperture 372 can be positioned over at least a portion of the aperture 828 in the absorbent layer 822 to allow access and fluid communication to at least a portion of the absorbent layers 822 and 851, transmission layer 811, and would contact layer 810 positioned below. The wound contact layer, the transmission layer, and/or the absorbent layer can be optional layers and the wound dressing can be formed without any of these layers.

An electronics assembly can be positioned in the apertures 828, 829, and 872 of the first and second absorbent material 851 and 822 and the cover layer 813. The electronics assembly can include a pump, power source, and a printed circuit board as described with reference to Figures 4A-5B, 6, and 7A-7D.

Before use, the dressing can include one or more delivery layers 846 adhered to the bottom surface of the wound contact layer. The delivery layer 846 can cover adhesive or apertures on the bottom surface of the wound contact layer 810. **In** some embodiments, the delivery layer 846 can provided support for the dressing and can assist in sterile and appropriate placement of the dressing over the wound and skin of the patient. The delivery layer 846 can include handles that can be used by the user to separate the delivery layer 846 from the wound contact layer 810 before applying the dressing to a wound and skin of a patient.

Figure 9 illustrates an embodiment of a wound dressing incorporating an electronics assembly 900 within the wound dressing layers 990. The electronics assembly 900 can be provided within the aperture 872 in the cover layer and apertures 829 and 828 in the first and second absorbent layers. In some embodiments, the electronics assembly 900 can seal to the outer perimeter of the aperture 872 of the cover layer.

The electronics assembly 900 can include the pump inlet protection mechanism extending from and sealed to the film as described in Figures 6 and 7A-7D. The electronics assembly 900 can be positioned within the apertures 872, 829, 828 in the cover layer and absorbent layer(s) as shown in Figure 9. In some embodiments, the perimeter of the electronics assembly 900 can be sealed to a top surface of the outer perimeter of the aperture 872 in the cover layer as shown in Figure 9. In some embodiments, the electronics assembly 700 is sealed to the cover layer 813 with a sealant gasket, adhesive, heat welding, adhesive bonding, ultrasonic welding, RF welding, or any other attachment or bonding technique. In some embodiments, the electronics assembly 900 can be permanently sealed to the cover layer 813 and could not be removed from the cover layer without destroying the dressing.

In some embodiments, the electronics assembly 900 can be utilized in a single dressing and disposed of with the dressing. In other embodiments, the electronics assembly 900 can be utilized or re-used (for example, after sterilization) in a series of dressings.

In some embodiments, the small apertured absorbent layer 851 can be positioned on top of the large apertured absorbent layer 822. In other embodiments, the small apertured absorbent layer 851 can be positioned below of the large apertured absorbent layer 822.

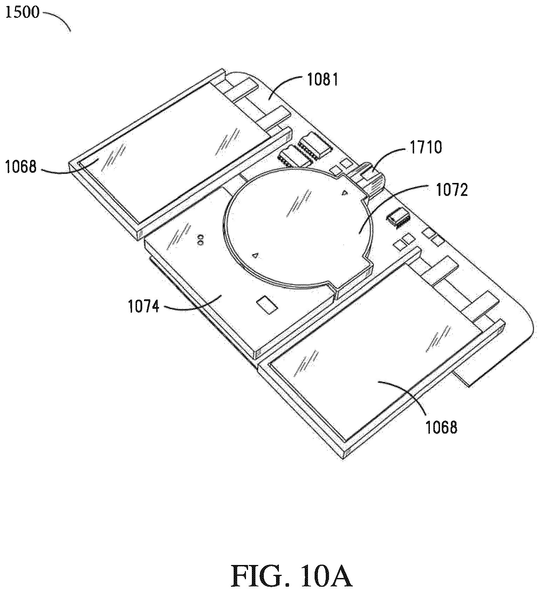

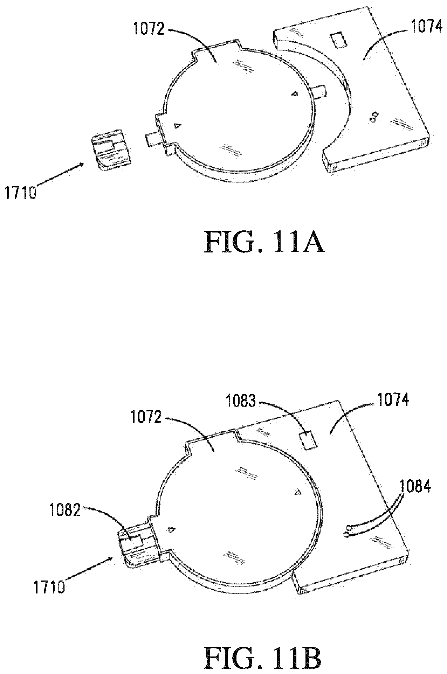

Figures 10A-10B and 11A-11B illustrate an electronics assembly 1500 with a pump inlet protection mechanism 1710 and pump exhaust mechanism 1074 on a pump 1072. The assembly 1500 can include cavities 1082 and 1083 (shown in Figures 11A-11B) on the pump inlet protection mechanism 1710 and pump exhaust mechanism 1074, respectively. In some embodiments, the inlet protection and pump exhaust mechanisms can be adhered to the inlet and the outlet of the pump as described herein. In some embodiments, the assembly 1500 can be assembled using an adhesive and allowed to cure prior to incorporating into the electronics assembly.

The pump inlet can be covered or fitted with a pump inlet protection mechanism 1710. In some embodiments, the pump inlet protection 1710 can be pushed onto the pump inlet as illustrated by the arrows in Figure 11A. This can be a friction fit. The port of the pump inlet protection 1710 that receives a portion of the pump inlet can be sized and shaped to be a complementary fit around the pump inlet. In some embodiments, the pump inlet protection 1710 can be bonded onto the pump inlet using a silicone sealant or any other sealant or sealing technique. Figure 11B illustrates the pump inlet protection mechanism 1710 covering the pump inlet and the pump exhaust mechanism 1074 covering the pump outlet. The pump exhaust mechanism 1074 can include one or more apertures or vents 1084 to allow gas aspirated by the pump to be exhausted from the pump exhaust mechanism 1074. In some cases, a non-return valve and/or filter membrane of the pump exhaust mechanism is included in the pump exhaust mechanism 1074.

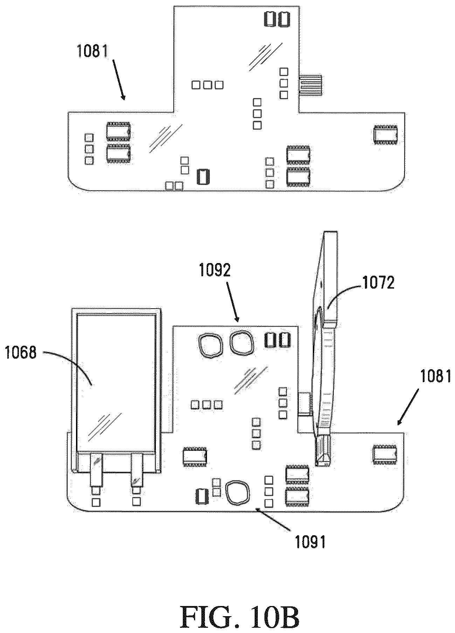

Figures 11A-11B illustrate the pump inlet protection mechanism 1710 and pump exhaust mechanism 1074 with cavities 1082 and 1083. A pump assembly including the pump inlet protection mechanism 1710 and pump exhaust mechanism 1074 can be placed over the surface of a circuit board 1081. When the pump assembly is in contact with the surface of the circuit board 1081, the cavities 1082 and 1083 can at least partially enclose sensors on the circuit board 1081, for example, pressure sensors 1091 and 1092 on the circuit board 1081, as illustrated in Figure 10B.

The pressure sensors 1091 and 1902 illustrated in Figure 10B can be used to measure and/or monitor the pressure level at the wound and atmospheric pressure. The pressure sensor 1091 can be used to measure and/or monitor pressure at the wound (such as, underneath the wound dressing), which can be accomplished by measuring and/or monitoring pressure in a fluid flow path connecting the negative pressure source or pump 1072 and the wound. In some embodiments, the pressure sensor 1091 can measure and/or monitor pressure in the cavity 1082 of the pump inlet protection mechanism 1710 shown in Figures 11A-11B.

The pressure sensor 1092 can be used to measure and/or monitor pressure external to the wound dressing. The pressure sensor 1092 can measure and/or monitor pressure in the cavity 1083 of the pump exhaust mechanism 1074 shown in Figures 11A-11B. The pressure sensor 1092 can measure pressure external to the wound dressing, which can be relative atmospheric pressure since the atmospheric pressure varies depending on, for instance, an altitude of use or pressurized environment in which the TNP apparatus may be used. These measurements can be used to establish a desired negative pressure differential (or set point) at the wound relative to the external pressure.

The circuit board 1081 (including any of the circuit boards described herein) can include control circuitry, such as one or more processors or controllers, that can control the supply of negative pressure by the negative pressure source 1072 according at least to a comparison between the pressure monitored by the pressure sensor 1091 and the pressure monitored by the pressure sensor 1092. Control circuity can operate the negative pressure source 1072 in a first mode (that can be referred to as an initial pump down mode) in which the negative pressure source 1072 is activated to establish the negative pressure set point at the wound. The set point can be set to, for example, a value in the range between about -70 mmHg to about -90 mmHg, among others. Once the set point has been established, which can be verified based on a difference between pressure measured by the pressure sensor 1091 (or wound pressure) and pressure measured by the pressure sensor 1092 (or external pressure), control circuitry can deactivate (or pause) operation of the negative pressure source 1072. Control circuitry can operate the negative pressure source 1072 is a second mode (that can be referred to as maintenance pump down mode) in which the negative pressure source 1072 is periodically activated to re-establish the negative pressure set point when the wound is depressurized as a result of one or more leaks. Control circuitry can activate the negative pressure source 1072 in response to the pressure at the wound (as monitored by the pressure sensor 1091) becomes more positive than a negative pressure threshold, which can be set to the same negative pressure as the set point or lower negative pressure.

Embodiments of the wound dressings, wound treatment apparatuses and methods described herein may also be used in combination or in addition to one or more features described in PCT International Application No. PCT/EP2017/060464, filed May 3, 2017, titled NEGATIVE PRESSURE WOUND THERAPY DEVICE ACTIVATION AND CONTROL, U.S. Patent No. 8,734,425, and U.S. Patent No. 8,905,985.

In some embodiments, one or more self-adhesive gaskets can be applied to the pump inlet protection mechanism 1710 and pump exhaust mechanism 1074 to seal the cavities 1082 and 1083 of the pump inlet and pump exhaust around sensors on the circuit board 1081 and to seal around the exhaust mechanism vent(s) and corresponding vent(s) in the circuit board 1081 (as described herein). In some embodiments, a pre-formed adhesive sheet can be used to form the sealing gaskets between the cavities 1082 and 1083 of the pump inlet and pump exhaust mechanisms and sensors on the circuit board 1081 and between the exhaust mechanism vent(s) and vent(s) in the circuit board 1081. In other embodiments, an adhesive can be used to seal the cavities 1082 and 1083 of the pump inlet protection 1710 and pump exhaust mechanism 1074 around sensors on the circuit board 1081 and to seal around the exhaust mechanism vent(s) 1084 and corresponding vent(s) in the circuit board. As described herein, the electronics assembly 1500 can be embedded within layers of the dressing, such as in cutouts or recesses into which the electronics assembly can be placed.

The pump inlet protection mechanism 1710 can provide a large surface area available for vacuum to be drawn by the inlet of the pump. A pump inlet (shown as rounded protrusion in Figure 11A) can fit within a recess in the pump inlet protection mechanism 1710. The pump inlet can be attached by friction fit and/or form a complementary fit to the recess of the pump inlet protection mechanism.

The pump inlet protection mechanism 1710 can allow air or gas to pass through, but can block liquid from reaching the negative pressure source. The pump inlet protection mechanism 1710 can include a porous material. The pump inlet protection mechanism 1710 can comprise one or more porous polymer molded components. The pump inlet protection mechanism 1710 can include hydrophobic or substantially hydrophobic material. In some embodiments, material included in the pump inlet protection mechanism 1710 can have a pore size in the range of approximately 5 microns to approximately 40 microns. In some embodiments, the pore size can be approximately 10 microns. In some embodiments, the pump inlet protection mechanism 1710 can include a polymer that can be one of hydrophobic polyethylene or hydrophobic polypropylene. In some embodiments, the pump inlet protection mechanism can include a Porvair Vyon material with a pore size of 10 microns. Any of the pump inlet protection mechanism described herein can include one or more features of the pump inlet protection mechanism 1710.

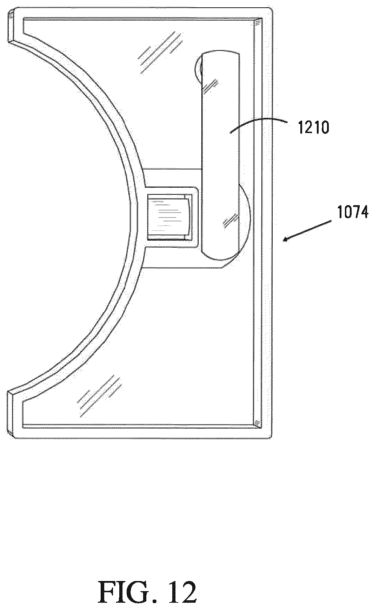

The pump exhaust mechanism 1074 (or any of the pump exhaust or outlet mechanisms described herein) can include a check valve or a non-return valve 1210 as shown in Figure 12. The non-return valve 1210 can be any suitable mechanical one-way valve, such as, for example, a reed valve, a duckbill valve, a ball valve, a loose leaf valve or an umbrella valve, among others. The non-return valve can be similar to any of the non-return valves described in PCT International Application No. PCT/EP2017/055225, filed March 6, 2017, titled WOUND TREATMENT APPARATUSES AND METHODS WITH NEGATIVE PRESSURE SOURCE INTEGRATED INTO WOUND DRESSING.

The pump exhaust mechanism 1074 can be bonded to the outlet of the pump using a sealant, for example a silicone sealant. The outlet or exhaust of the pump exhaust mechanism 1074 can include an antimicrobial film and/or other filter membrane that filters gas exhausted outside the NPWT system, such as to the atmosphere. As illustrated, pump exhaust mechanism 1074 can be an enclosure or chamber that is substantially sealed to prevent ingress of gas or fluid other than through the vent(s) 1084.

Any of the embodiments described herein can additionally or alternatively include one or more features described in International Application No. PCT/EP2018/074694, filed September 13, 2018, titled NEGATIVE PRESSURE WOUND TREATMENT APPARATUSES AND METHODS WITH INTEGRATED ELECTRONICS, International Application No. PCT/EP2018/074701, filed September 13, 2018, titled NEGATIVE PRESSURE WOUND TREATMENT APPARATUSES AND METHODS WITH INTEGRATED ELECTRONICS, and International Application No. PCT/EP2018/074694, filed October 25, 2018, titled NEGATIVE PRESSURE WOUND TREATMENT APPARATUSES AND METHODS WITH INTEGRATED ELECTRONICS.

### Exhaust/Downstream Blockage Detection

In operation, one or more vents in pump exhaust mechanism can become blocked. For example, a user of the NPWT system can sit or lay on the dressing and/or the dressing can be incorrectly applied so as to block the one or more vents. Blockage of the one or more vents can undesirably cause system malfunction, such as incorrect operation, inefficient use of power supplied by the power source, or the like. It can be advantageous to detect blockage of the one or more vents of the pump exhaust mechanism (and/or any of the vents in the other components of the NPWT systems that are in fluidic connection with such vents, as described herein) or, generally, to detect blockage downstream of the negative pressure source. Once detected, indication of such blockage (which can be referred to as downstream blockage or exhaust blockage) can be provided to a user to facilitate remedying the blockage. Such indication can be provided visually, audibly, tactilely, and/or via communication to a remote computing device, among others. Any of the indicators described herein can be used for providing the downstream blockage indication. In some cases, indication of downstream blockage can include deactivating the source of negative pressure.

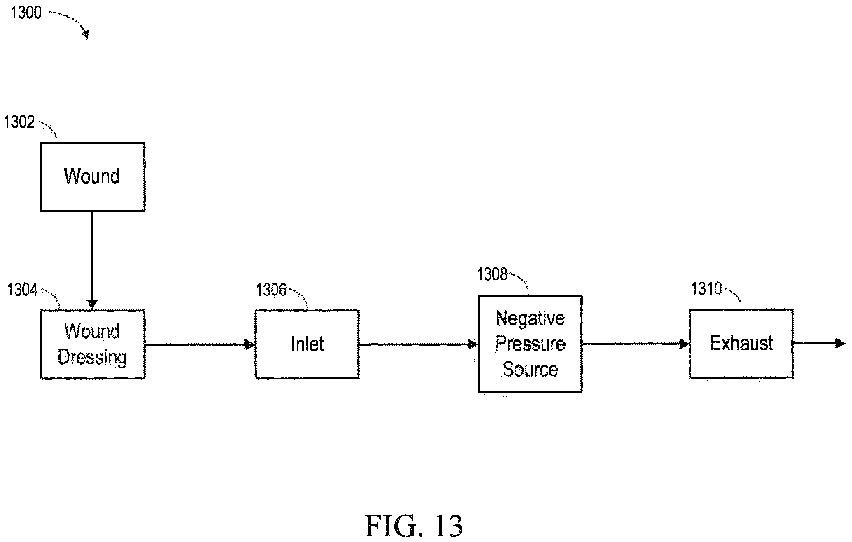

Figure 13 illustrates a fluid flow path 1300 of an NPWT system, including any of the systems described herein. The fluid flow path 1300 includes a wound 1302, wound dressing 1304 covering the wound 1302, a negative pressure source 1308 with an inlet 1306, and exhaust 1310 (which can be any of the exhaust mechanisms described herein). Fluid flows through the fluid flow path 1300 from left to right. Downstream blockage can occur in any portion of the fluid flow path 1300 to the right (or downstream) of the negative pressure source 1308.

In some cases, downstream blockage can cause an increase in pressure in or within the pump exhaust mechanism, which as described herein can be a substantially sealed chamber (except for the one or more vents). Such rise in pressure can be caused by accumulation in the pump exhaust mechanism chamber of gas aspirated by the negative pressure source from the wound. If the blockage is not detected, the system can erroneously determine that, based on pressure differential between wound pressure (for example, measured by the pressure sensor 1091) and the incorrectly measured external pressure and pressure (for example, measured by the pressure sensor 1092), set point negative pressure has been established at the wound, when in fact it has not been established. Downstream blockage can be detected by using a pressure sensor that measures pressure in the pump exhaust mechanism, such as the pressure sensor 1092 described herein with reference to Figures 10-11. Pressure measured in or within the pump exhaust mechanism can be compared by the system (for example, by control circuitry) to a downstream blockage threshold. If the measured pressure satisfies the threshold, downstream blockage can be detected and/or indicated.

In some instances, the downstream pressure threshold corresponds to external pressure, which can be atmospheric pressure (such as the relative atmospheric pressure). Atmospheric pressure measured by the pressure sensor (for example, pressure sensor 1092) when the system initiates (for example, via control circuitry) negative pressure wound therapy can be recorded. For example, atmospheric pressure can be recorded before (or soon after) the negative pressure source 1072 is operated in the initial pump down mode, in which the negative pressure source is activated to establish the negative pressure set point at the wound, as described herein. Advantageously, recording atmospheric pressure at such time can increase accuracy as it would be expected that the pressure in the pump exhaust mechanism has been substantially equated with the atmospheric pressure during the time the system has not been providing therapy.

In some instances, downstream pressure threshold recorded as described above can be adjusted by an offset. The offset can be a positive pressure offset, such as about 25%, about 30%, or about 40% of the recorded pressure value, among others, a fixed pressure value, such as, about 10 mmHg, about 20 mmHg, about 30 mmHg, among others, or an adjustable value. Using the offset can improve accuracy of detecting downstream blockages by, among others, minimizing or preventing false positive events, minimizing or preventing detection and/or indication of transient downstream blockages that may quickly clear, or the like.

After setting the downstream pressure threshold, the system (for example, control circuitry) can determine if a downstream blockage is present. When the negative pressure source is activated, the system (for example, control circuitry) can obtain current pressure in or within the pump exhaust mechanism and compare the current pressure to the downstream pressure threshold. If the threshold is satisfied (such as, met or exceeded), the system (for example, control circuitry) can determine that the downstream blockage is present. Indication of downstream blockage can be provided. Determination of whether the downstream blockage is present can be performed periodically. In some cases, the determination can be performed following activation of the negative pressure source in the initial pump down mode and/or maintenance pump down mode. The determination can be performed following each activation of the negative pressure source.

In some implementations, pressure in the pump exhaust mechanism can be output by the pressure sensor as a voltage (or current), rather than an absolute pressure measurement. For example, the pressure sensor can provide a voltage output in a range of about 0.2V to about 4.7V, which can correspond to pressure in a range of about 0mmHg to about 860 mmHg (or about 1151.5 mbar). External pressure (for example, atmospheric pressure at sea level) can be about 767 mmHg (or about 1023 mbar), and the offset can be about 22.5 mmHg (or about 30 mbar). Thus, the downstream pressure threshold can be set to about 789.5mmHg (or about 1053 mbar). In this example, pressure in the pump exhaust mechanism that satisfies and/or exceeds about 789.5 mmHg, which can correspond to about 4.3V, can be indicative of downstream blockage.

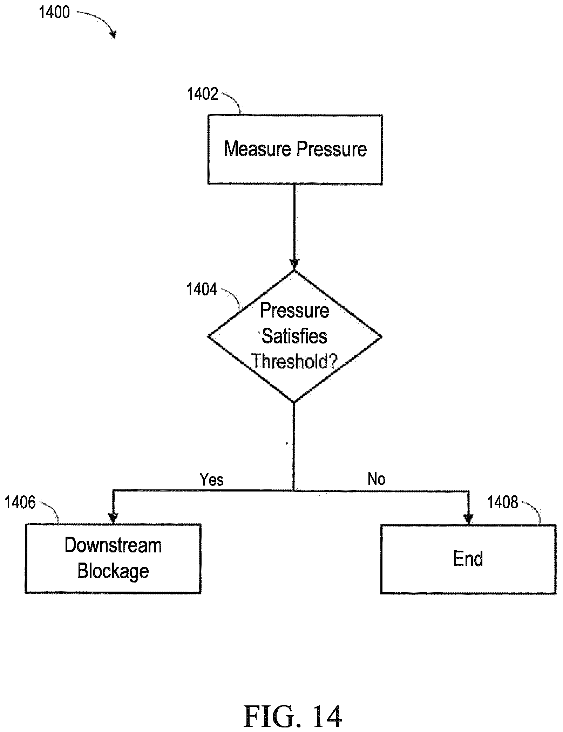

Figure 14 illustrates a process 1400 for detecting downstream blockages. The process 1400 can be implemented by control circuitry of an NPWT system, including any of the systems described herein. In block 1402, the process 1400 receives current pressure measurement in or within the pump exhaust mechanism from the pressure sensor, as described herein. In block 1404, the process 1400 compares the pressure measurement to a downstream blockage threshold, previously set as described herein. If the downstream blockage threshold is satisfied, the process 1400 transitions to block 1406 associated with determination and/or indication of downstream blockage. If in block 1404 the process 1400 determines that the downstream blockage threshold is not satisfied, the process terminates in block 1408.

While certain embodiments described herein relate to integrated negative pressure wound therapy systems in which the negative pressure source is supported by the dressing, systems and methods described herein are applicable to any negative pressure wound therapy system or medical systems. For example, blockage detection systems and methods described herein can be used in fluid-proof (such as, water-proof) negative pressure wound therapy systems or medical systems. As described herein, such systems can be configured to exhaust gas from one or more apertures or vents of an exhaust mechanism (which can be a chamber within a housing of the system or entire housing), while the remainder of the exhaust mechanism is sealed or substantially sealed. Blockage of the one or more vents can cause system malfunction and such blockages can be detected and/or indicated, as described herein. More generally, blockages downstream of the negative pressure source can be detected and/or indicated.

### Upstream Blockage Detection

In some cases, one or more blockages upstream of a negative pressure source, such as in any portion of the fluid flow path 1300 to the left (or upstream) of the negative pressure source 1308 in Figure 13 can additionally (or alternatively) be determined and/or indicated. Such blockages can be referred to as upstream blockages, and may be due to, for instance, the wound dressing being saturated or substantially saturated with fluid aspirated from the wound. This, in turn, can cause the pump inlet protection mechanism to at least partially block fluid flow to the negative pressure source, which can cause decrease (or loss) in negative pressure at the wound. It can be advantageous to detect and/or indicate upstream blockages to facilitate provision of negative pressure wound therapy with minimal interruptions or without interruptions, to facilitate safe provision of negative pressure wound therapy, and the like.

The system (for example, control circuitry) can determine presence of an upstream blockage by comparing pressure at the wound (for example, measured by the pressure sensor 1091) to an upstream blockage threshold. If the threshold is satisfied, presence of upstream blockage and be determined and/or indicated. Upstream blockage threshold can be determined and/or set to pressure associated with a particular level of fluid in the dressing, such as expected loss of pressure caused by the dressing having absorbed the particular amount of fluid. For example, the upstream blockage threshold can be associated with full or substantially full dressing, 95% full dressing, 90% full dressing, 85% full dressing, or the like. In some cases, partial or full blockage of the inlet protection mechanism, such as of the filter in the inlet protection mechanism, can be determined and/or indicated. Indication of the upstream blockage can be provided using any of the approaches described herein.

### Other Variations

Although certain embodiments described herein relate to wound dressings, systems and methods disclosed herein are not limited to wound dressings or medical applications. Systems and methods disclosed herein are generally applicable to electronic devices in general, such as electronic devices that can be worn by or applied to a user.

Any value of a threshold, limit, duration, etc. provided herein is not intended to be absolute and, thereby, can be approximate. In addition, any threshold, limit, duration, etc. provided herein can be fixed or varied either automatically or by a user. Furthermore, as is used herein relative terminology such as exceeds, greater than, less than, etc. in relation to a reference value is intended to also encompass being equal to the reference value. For example, exceeding a reference value that is positive can encompass being equal to or greater than the reference value. In addition, as is used herein relative terminology such as exceeds, greater than, less than, etc. in relation to a reference value is intended to also encompass an inverse of the disclosed relationship, such as below, less than, greater than, etc. in relations to the reference value. Moreover, although blocks of the various processes may be described in terms of determining whether a value meets or does not meet a particular threshold, the blocks can be similarly understood, for example, in terms of a value (i) being below or above a threshold or (ii) satisfying or not satisfying a threshold.

While certain embodiments have been described, these embodiments have been presented by way of example only, and are not intended to limit the scope of protection. Indeed, the novel methods and systems described herein may be embodied in a variety of other forms. Furthermore, various omissions, substitutions and changes in the form of the methods and systems described herein may be made. Those skilled in the art will appreciate that in some embodiments, the actual steps taken in the processes illustrated or disclosed may differ from those shown in the figures. Depending on the embodiment, certain of the steps described above may be removed, others may be added. For example, the actual steps or order of steps taken in the disclosed processes may differ from those shown in the figure.

Depending on the embodiment, certain of the steps described above may be removed, others may be added. For instance, the various components illustrated in the figures may be implemented as software or firmware on a processor, controller, ASIC, FPGA, or dedicated hardware. Hardware components, such as controllers, processors, ASICs, FPGAs, and the like, can include logic circuitry.

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount.

## Claims

1. A negative pressure wound therapy system comprising:
a wound dressing configured to be placed over a wound, the wound dressing configured to absorb fluid;
a source of negative pressure (1072) disposed on or within the wound dressing, the source of negative pressure configured to aspirate fluid from the wound;
a vent (1084) configured to allow gas aspirated by the source of negative pressure to flow outside the system;
a pressure sensor (1092) at least partially disposed on or within the wound dressing proximal to the vent; and
a controller disposed on or within the wound dressing, **characterized in that** the controller is configured to:
in response to a determination that pressure measured by the pressure sensor satisfies a threshold indicative of blockage in the vent, provide an indication that the vent is blocked.

2. The system of claim 1, further comprising a chamber at least partially disposed on or within the wound dressing, the chamber housing the pressure sensor and comprising the vent through a wall of the chamber.

3. The system of claim 1 or claim 2, wherein the controller is further configured to set the threshold to a value associated with an initial pressure measured by the pressure sensor prior to activation of the source of negative pressure; optionally,
wherein the controller is further configured to adjust the set threshold by a positive pressure offset.

4. The system of any one of the preceding claims, wherein the controller is further configured to:
periodically activate the source of negative pressure to establish a target negative pressure under the wound dressing; and
in response to establishing the target negative pressure under the wound dressing, deactivate the source of negative pressure.

5. The system of claim 4, wherein the controller is further configured to:
in response to a determination that pressure measured by the pressure sensor following an activation of the source of negative pressure satisfies the threshold, provide the indication that the vent is blocked; optionally,
wherein the controller is configured to determine if the pressure measured by the pressure sensor satisfies the threshold following each activation of the source of negative pressure.

6. The system of any one of the preceding claims, further comprising another pressure sensor configured to measure pressure in a fluid flow path connecting the source of negative pressure to the wound, wherein the controller is further configured to:
based on pressure measured by the another pressure sensor, determine if a level of fluid absorbed by the wound dressing satisfies a threshold fluid level; and
provide another indication in response to a determination that the level of fluid absorbed by the wound dressing satisfied the threshold fluid level; optionally,
wherein the threshold fluid level is indicative of the wound dressing being substantially full.

7. The system of any one of the preceding claims further comprising a filter positioned upstream of the source of negative pressure and configured to substantially prevent passage of fluid downstream of the filter, wherein the controller is further configured to determine blockage of the filter and provide another indication associated with the blockage.

8. The system of any one of the preceding claims further comprising at least one indicator positioned at least partially on an exterior surface of the wound dressing, wherein the controller is configured to provide at least one of the indication or another indication via the at least one indicator.

9. A negative pressure wound therapy system comprising:
a source of negative pressure (1072) configured to aspirate fluid from a wound covered by a wound dressing;
a first pressure sensor (1092) configured to measure pressure downstream of the source of negative pressure; and
a controller configured to:
in response to a determination that pressure measured by the first pressure sensor satisfies a threshold indicative of a first blockage downstream of the source of negative pressure, provide an indication of the first blockage; optionally,
wherein the system further comprising a vent (1084) configured to release gas aspirated by the source of negative pressure into the atmosphere, wherein the first blockage is associated with blockage of the vent.

10. The system of claim 9, further comprising an enclosure comprising a vent in communication with the atmosphere, the first sensor being positioned in the enclosure.

11. The system of claim 9 or claim 10, wherein the threshold is indicative of atmospheric pressure adjusted by a positive pressure offset.

12. The system of any one of claims 9 to 11, wherein the controller is further configured to detect and provide an indication of a second blockage upstream of the source of negative pressure.

13. The system of any one of claims 9 to 12, further comprising a second pressure sensor configured to measure pressure upstream of the source of negative pressure, wherein the controller is further configured to:
in response to a determination that pressure measured by the second pressure sensor satisfies a threshold indicative of a second blockage upstream of the source of negative pressure, provide an indication of the second blockage.

14. The system of claim 12 or claim 13, wherein the second blockage is associated with blockage in a fluid flow path connecting the source of negative pressure to the wound dressing.

15. The system of any one of claims 9 to 14 further comprising at least one indicator visible to a user, wherein the controller is configured to provide at least one of the indications of the first or second blockages via the at least one indicator.

## Patentansprüche

1. Ein Unterdruck-Wundtherapiesystem, das Folgendes beinhaltet:
einen Wundverband, der konfiguriert ist, um über einer Wunde platziert zu werden, wobei der Wundverband konfiguriert ist, um Fluid zu absorbieren;
eine Unterdruckquelle (1072), die auf oder in dem Wundverband angeordnet ist, wobei die Unterdruckquelle konfiguriert ist, um Fluid aus der Wunde abzusaugen;
eine Lüftungsöffnung (1084), die konfiguriert ist, um zu ermöglichen, dass durch die Unterdruckquelle angesaugtes Gas nach außerhalb des Systems fließt,
einen Drucksensor (1092), der proximal der Lüftungsöffnung mindestens teilweise auf oder in dem Wundverband angeordnet ist; und
eine Steuereinrichtung, die auf oder in dem Wundverband angeordnet ist, **dadurch gekennzeichnet, dass** die Steuereinrichtung für Folgendes konfiguriert ist:
als Reaktion auf eine Bestimmung, dass ein durch den Drucksensor gemessener Druck einem Schwellenwert genügt, der für eine Verstopfung in der Lüftungsöffnung indikativ ist, Bereitstellen eines Hinweises, dass die Lüftungsöffnung verstopft ist.

2. System gemäß Anspruch 1, das ferner eine Kammer beinhaltet, die mindestens teilweise auf oder in dem Wundverband angeordnet ist, wobei die Kammer den Drucksensor beherbergt und die Lüftungsöffnung durch eine Wand der Kammer beinhaltet.

3. System gemäß Anspruch 1 oder Anspruch 2, wobei die Steuereinrichtung ferner konfiguriert ist, um den Schwellenwert auf einen Wert zu setzen, der mit einem durch den Drucksensor vor der Aktivierung der Unterdruckquelle gemessenen Anfangsdruck assoziiert ist;
wobei optional die Steuereinrichtung ferner konfiguriert ist, um den gesetzten Schwellenwert durch eine Überdruckverschiebung anzupassen.

4. System gemäß einem der vorhergehenden Ansprüche, wobei die Steuereinrichtung ferner für Folgendes konfiguriert ist:
periodisches Aktivieren der Unterdruckquelle, um einen Zielunterdruck unter dem Wundverband herzustellen; und
als Reaktion auf das Herstellen des Zielunterdrucks unter dem Wundverband, Deaktivieren der Unterdruckquelle.

5. System gemäß Anspruch 4, wobei die Steuereinrichtung ferner für Folgendes konfiguriert ist:
als Reaktion auf eine Bestimmung, dass ein durch den Drucksensor im Anschluss an eine Aktivierung der Unterdruckquelle gemessener Druck dem Schwellenwert genügt, Bereitstellen eines Hinweises darauf, dass die Lüftungsöffnung verstopft ist;
wobei optional die Steuereinrichtung konfiguriert ist, um im Anschluss an jede Aktivierung der Unterdruckquelle zu bestimmen, ob der durch den Drucksensor gemessene Druck dem Schwellenwert genügt.

6. System gemäß einem der vorhergehenden Ansprüche, das ferner einen weiteren Drucksensor beinhaltet, der konfiguriert ist, um in einem Fluidfließweg, der die Unterdruckquelle mit der Wunde verbindet, Druck zu messen, wobei die Steuereinrichtung ferner für Folgendes konfiguriert ist:
basierend auf einem durch den weiteren Drucksensor gemessenen Druck, Bestimmen, ob eine Menge eines durch den Wundverband absorbierten Fluids einer Schwellenwertfluidmenge genügt, und
Bereitstellen eines weiteren Hinweises als Reaktion auf eine Bestimmung, dass die Menge des durch den Wundverband absorbierten Fluids der Schwellenwertfluidmenge entspricht;
wobei optional die Schwellenwertfluidmenge dafür indikativ ist, dass der Wundverband im Wesentlichen voll ist.

7. System gemäß einem der vorhergehenden Ansprüche, das ferner einen Filter beinhaltet, der stromaufwärts der Unterdruckquelle positioniert und konfiguriert ist, um einen Durchgang von Fluid nach stromabwärts des Filters im Wesentlichen zu verhindern, wobei die Steuereinrichtung ferner konfiguriert ist, um eine Verstopfung des Filters zu bestimmen und einen mit der Verstopfung assoziierten weiteren Hinweis bereitzustellen.

8. System gemäß einem der vorhergehenden Ansprüche, das ferner mindestens einen Indikator beinhaltet, der mindestens teilweise auf einer äußeren Oberfläche des Wundverbands positioniert ist, wobei die Steuereinrichtung konfiguriert ist, um mindestens einen von dem Hinweis oder dem weiteren Hinweis mittels des mindestens einen Indikators bereitzustellen.

9. Ein Unterdruck-Wundtherapiesystem, das Folgendes beinhaltet:
eine Unterdruckquelle (1072), die konfiguriert ist, um Fluid aus einer mit einem Wundverband abgedeckten Wunde abzusaugen;
einen ersten Drucksensor (1092), der konfiguriert ist, um stromabwärts der Unterdruckquelle Druck zu messen; und
eine Steuereinrichtung, die für Folgendes konfiguriert ist:
als Reaktion auf eine Bestimmung, dass ein durch den ersten Drucksensor gemessener Druck einem Schwellenwert genügt, der für eine erste Verstopfung stromabwärts der Unterdruckquelle indikativ ist, Bereitstellen eines Hinweises auf die erste Verstopfung;
wobei optional das System ferner eine Lüftungsöffnung (1084) beinhaltet, die konfiguriert ist, um durch die Unterdruckquelle angesaugtes Gas in die Atmosphäre freizusetzen, wobei die erste Verstopfung mit einer Verstopfung der Lüftungsöffnung assoziiert ist.

10. System gemäß Anspruch 9, das ferner eine Umhüllung beinhaltet, die eine Lüftungsöffnung in Kommunikation mit der Atmosphäre beinhaltet, wobei der erste Sensor in der Umhüllung positioniert ist.

11. System gemäß Anspruch 9 oder Anspruch 10, wobei der Schwellenwert für durch eine Überdruckverschiebung angepassten atmosphärischen Druck indikativ ist.

12. System gemäß einem der Ansprüche 9 bis 11, wobei die Steuereinrichtung ferner konfiguriert ist, um eine zweite Verstopfung stromaufwärts der Unterdruckquelle zu detektieren und einen Hinweis darauf bereitzustellen.

13. System gemäß einem der Ansprüche 9 bis 12, das ferner einen zweiten Drucksensor beinhaltet, der konfiguriert ist, um stromaufwärts der Unterdruckquelle Druck zu messen, wobei die Steuereinrichtung ferner für Folgendes konfiguriert ist:
als Reaktion auf eine Bestimmung, dass ein durch den zweiten Drucksensor gemessener Druck einem Schwellenwert genügt, der für eine zweite Verstopfung stromaufwärts der Unterdruckquelle indikativ ist, Bereitstellen eines Hinweises auf die zweite Verstopfung.

14. System gemäß Anspruch 12 oder Anspruch 13, wobei die zweite Verstopfung mit einer Verstopfung in einem Fluidfließweg, der die Unterdruckquelle mit dem Wundverband verbindet, assoziiert ist.

15. System gemäß einem der Ansprüche 9 bis 14, das ferner mindestens einen für einen Benutzer sichtbaren Indikator beinhaltet, wobei die Steuereinrichtung konfiguriert ist, um mindestens einen der Hinweise auf die erste oder die zweite Verstopfung mittels des mindestens einen Indikators bereitzustellen.

## Revendications

1. Un système de thérapie des plaies par pression négative comprenant :
un pansement pour plaie configuré pour être placé par-dessus une plaie, le pansement pour plaie étant configuré pour absorber du fluide ;
une source de pression négative (1072) disposée sur ou à l'intérieur du pansement pour plaie, la source de pression négative étant configurée pour aspirer du fluide provenant de la plaie ;
un évent (1084) configuré pour permettre à du gaz aspiré par la source de pression négative de s'écouler vers l'extérieur du système ;
un capteur de pression (1092) au moins partiellement disposé sur ou à l'intérieur du pansement pour plaie à proximité de l'évent ; et
un organe de commande disposé sur ou à l'intérieur du pansement pour plaie,
**caractérisé en ce que** l'organe de commande est configuré pour :
en réponse à une détermination qu'une pression mesurée par le capteur de pression satisfait à un seuil indicatif d'une obstruction dans l'évent, fournir une indication que l'évent est obstrué.

2. Le système de la revendication 1, comprenant en outre une chambre au moins partiellement disposée sur ou à l'intérieur du pansement pour plaie, la chambre logeant le capteur de pression et comprenant l'évent à travers une paroi de la chambre.

3. Le système de la revendication 1 ou de la revendication 2, où l'organe de commande est configuré en outre pour fixer le seuil sur une valeur associée à une pression initiale mesurée par le capteur de pression avant activation de la source de pression négative ; facultativement
où l'organe de commande est configuré en outre pour ajuster le seuil fixé par un décalage de pression positive.

4. Le système de n'importe laquelle des revendications précédentes, où l'organe de commande est configuré en outre pour :
activer périodiquement la source de pression négative afin d'établir une pression négative cible en dessous du pansement pour plaie ; et
en réponse à l'établissement de la pression négative cible en dessous du pansement pour plaie, désactiver la source de pression négative.

5. Le système de la revendication 4, où l'organe de commande est configuré en outre pour :
en réponse à une détermination qu'une pression mesurée par le capteur de pression à la suite d'une activation de la source de pression négative satisfait au seuil, fournir l'indication que l'évent est obstrué ; facultativement,
où l'organe de commande est configuré pour déterminer si la pression mesurée par le capteur de pression satisfait au seuil à la suite de chaque activation de la source de pression négative.

6. Le système de n'importe laquelle des revendications précédentes, comprenant en outre un autre capteur de pression configuré pour mesurer une pression dans une voie d'écoulement de fluide raccordant la source de pression négative à la plaie, où l'organe de commande est configuré en outre pour :
sur la base d'une pression mesurée par cet autre capteur de pression, déterminer si un niveau de fluide absorbé par le pansement pour plaie satisfait à un niveau de fluide seuil ; et
fournir une autre indication en réponse à une détermination que le niveau de fluide absorbé par le pansement pour plaie satisfaisait au niveau de fluide seuil ;
facultativement,
où le niveau de fluide seuil est indicatif du fait que le pansement pour plaie est substantiellement plein.

7. Le système de n'importe laquelle des revendications précédentes comprenant en outre un filtre positionné en amont de la source de pression négative et configuré pour empêcher substantiellement le passage de fluide en aval du filtre, où l'organe de commande est configuré en outre pour déterminer une obstruction du filtre et fournir une autre indication associée à l'obstruction.

8. Le système de n'importe laquelle des revendications précédentes comprenant en outre au moins un indicateur positionné au moins partiellement sur une surface extérieure du pansement pour plaie, où l'organe de commande est configuré pour fournir cette indication et/ou une autre indication par le biais de l'au moins un indicateur.

9. Un système de thérapie des plaies par pression négative comprenant :
une source de pression négative (1072) configurée pour aspirer du fluide provenant d'une plaie recouverte par un pansement pour plaie ;
un premier capteur de pression (1092) configuré pour mesurer une pression en aval de la source de pression négative ; et
un organe de commande configuré pour :
en réponse à une détermination qu'une pression mesurée par le premier capteur de pression satisfait à un seuil indicatif d'une première obstruction en aval de la source de pression négative, fournir une indication de la première obstruction ; facultativement, le système comprenant en outre un évent (1084) configuré pour relâcher du gaz aspiré par la source de pression négative dans l'atmosphère, où la première obstruction est associée à une obstruction de l'évent.

10. Le système de la revendication 9, comprenant en outre une enceinte comprenant un évent en communication avec l'atmosphère, le premier capteur étant positionné dans l'enceinte.

11. Le système de la revendication 9 ou de la revendication 10, où le seuil est indicatif d'une pression atmosphérique ajustée par un décalage de pression positive.

12. Le système de n'importe laquelle des revendications 9 à 11, où l'organe de commande est configuré en outre pour détecter et fournir une indication d'une deuxième obstruction en amont de la source de pression négative.

13. Le système de n'importe laquelle des revendications 9 à 12 comprenant en outre un deuxième capteur de pression configuré pour mesurer une pression en amont de la source de pression négative, où l'organe de commande est configuré en outre pour : en réponse à une détermination qu'une pression mesurée par le deuxième capteur de pression satisfait à un seuil indicatif d'une deuxième obstruction en amont de la source de pression négative, fournir une indication de la deuxième obstruction.

14. Le système de la revendication 12 ou de la revendication 13, où la deuxième obstruction est associée à une obstruction dans une voie d'écoulement de fluide raccordant la source de pression négative au pansement pour plaie.

15. Le système de n'importe laquelle des revendications 9 à 14 comprenant en outre au moins un indicateur visible par un utilisateur, où l'organe de commande est configuré pour fournir au moins l'une des indications des première ou deuxième obstructions par le biais de l'au moins un indicateur.
